# EUROPEAN PATENT APPLICATION

(11) **EP 3 812 399 A1**
(43) Date of publication of application: **28.04.2021**
(21) Application number: 19822018.8
(22) Date of filing: 20.06.2019
(51) Int. Cl.: C07K 16/46, A61K 39/395, A61P 35/00, C07K 16/28, C07K 16/30

(54) **METHOD FOR ACTIVATING IMMUNE RESPONSE OF TARGET CELL AND COMPOSITION THEREFOR**

(30) Priority: 20.06.2018 JP 2018116978
(71) Applicant: CHUGAI SEIYAKU KABUSHIKI KAISHA, Tokyo 115-8543 (JP)
(72) Inventor: SAKASHITA, Takuya, Gotemba-shi, Shizuoka 412-8513 (JP); SAVORY, Nasa, Gotemba-shi, Shizuoka 412-8513 (JP); KATO, Kazuki, Gotemba-shi, Shizuoka 412-8513 (JP); NARUSHIMA, Yuta, Kamakura-shi, Kanagawa 247-8530 (JP); MURAKAMI, Ryuichi, Kamakura-shi, Kanagawa 247-8530 (JP); IGAWA, Tomoyuki, Gotemba-shi, Shizuoka 412-8513 (JP)
(74) Representative: Vossius & Partner Patentanwälte Rechtsanwälte mbB
(86) International application number: PCT/JP2019/024479
(87) International publication number: WO 2019/244973

(57) **Abstract**

In one embodiment, a multispecific antigen-binding molecule that recognizes an antigen on an antigen-presenting cell and an antigen on a target cell, which is capable of crosslinking the antigen-presenting cell and the target cell, is provided.

SEQUENCE LISTING

<110>  CHUGAI SEIYAKU KABUSHIKI KAISHA

<120>  METHOD AND COMPOSITION FOR ACTIVATING IMMUNE RESPONSE AGAINST
       TARGET CELLS

<130>  C1-A1805P

<150>  JP 2018-116978
<151>  2018-06-20

<160>  63

<170>  PatentIn version 3.5

<210>  1
<211>  442
<212>  PRT
<213>  Artificial sequence

<220>
<223>  An artificially synthesized sequence

<400>  1

Ala Val Gln Ile Val Glu Ser Gly Gly Gly Leu Val Gln Pro Lys Glu
1               5                   10                  15

Ser Leu Lys Ile Ser Cys Thr Ala Ser Gly Phe Thr Phe Ser Asn Ala
            20                  25                  30

Ala Ile Tyr Trp Val Arg Gln Thr Pro Gly Lys Gly Leu Glu Trp Val
        35                  40                  45

Gly Arg Ile Arg Thr Arg Pro Ser Lys Tyr Ala Thr Asp Tyr Ala Asp
    50                  55                  60

Ser Val Arg Gly Arg Phe Thr Ile Ser Arg Asp Asp Ser Lys Ser Met
65                  70                  75                  80

Val Tyr Leu Gln Met Asp Asn Leu Arg Thr Glu Asp Thr Ala Met Tyr
                85                  90                  95

Tyr Cys Thr Pro Arg Ala Thr Glu Asp Val Pro Phe Tyr Trp Gly Gln
            100                 105                 110

Gly Val Met Val Thr Val Ser Ser Ala Lys Thr Thr Pro Pro Ser Val
        115                 120                 125

Tyr Pro Leu Ala Pro Gly Ser Ala Ala Gln Thr Asn Ser Met Val Thr
    130                 135                 140

Leu Gly Cys Leu Val Lys Gly Tyr Phe Pro Glu Pro Val Thr Val Thr

145                 150                 155                 160

Trp Asn Ser Gly Ser Leu Ser Ser Gly Val His Thr Phe Pro Ala Val
                165                 170                 175

Leu Gln Ser Asp Leu Tyr Thr Leu Ser Ser Ser Val Thr Val Pro Ser
            180                 185                 190

Ser Thr Trp Pro Ser Glu Thr Val Thr Cys Asn Val Ala His Pro Ala
        195                 200                 205

Ser Ser Thr Lys Val Asp Lys Lys Ile Val Pro Arg Asp Cys Gly Cys
    210                 215                 220

Lys Pro Cys Ile Cys Thr Val Lys Glu Val Ser Lys Val Phe Ile Phe
225                 230                 235                 240

Pro Pro Lys Pro Lys Asp Val Leu Thr Ile Thr Leu Thr Pro Lys Val
                245                 250                 255

Thr Cys Val Val Val Asp Ile Ser Lys Asp Asp Pro Glu Val Gln Phe
            260                 265                 270

Ser Trp Phe Val Asp Asp Val Glu Val His Thr Ala Gln Thr Gln Pro
        275                 280                 285

Arg Glu Glu Gln Phe Asn Ser Thr Phe Arg Ser Val Ser Glu Leu Pro
    290                 295                 300

Ile Met His Gln Asp Trp Leu Asn Gly Lys Glu Phe Lys Cys Arg Val
305                 310                 315                 320

Asn Ser Ala Ala Phe Pro Ala Pro Ile Glu Lys Thr Ile Ser Lys Thr
                325                 330                 335

Lys Gly Arg Pro Lys Ala Pro Gln Val Tyr Thr Ile Pro Pro Pro Lys
            340                 345                 350

Glu Gln Met Ala Lys Asp Lys Val Ser Leu Thr Cys Met Ile Thr Asp
        355                 360                 365

Phe Phe Pro Glu Asp Ile Thr Val Glu Trp Gln Trp Asn Gly Gln Pro
    370                 375                 380

Ala Glu Asn Tyr Lys Asn Thr Gln Pro Ile Met Arg Thr Asp Gly Ser
385                 390                 395                 400

Tyr Phe Val Tyr Ser Lys Leu Asn Val Gln Lys Ser Asn Trp Glu Ala
                405                 410                 415

Gly Asn Thr Phe Thr Cys Ser Val Leu His Glu Gly Leu His Asn His
            420                 425                 430

His Thr Glu Lys Ser Leu Ser His Ser Pro
        435                 440

<210>  2
<211>  220
<212>  PRT
<213>  Artificial sequence

<220>
<223>  An artificially synthesized sequence

<400>  2

Asp Ile Val Met Thr Gln Thr Pro Ser Ser Gln Ala Val Ser Ala Gly
1               5                   10                  15

Glu Lys Val Thr Met Asn Cys Lys Ser Ser Gln Ser Val Leu Tyr Asp
            20                  25                  30

Glu Asn Lys Lys Asn Tyr Leu Ala Trp Tyr Gln Gln Lys Ser Gly Gln
        35                  40                  45

Ser Pro Lys Leu Leu Ile Tyr Trp Ala Ser Thr Gly Glu Ser Gly Val
    50                  55                  60

Pro Asp Arg Phe Ile Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Gln
                85                  90                  95

Tyr Tyr Asp Phe Pro Pro Thr Phe Gly Gly Gly Thr Lys Leu Glu Leu
            100                 105                 110

Lys Arg Ala Asp Ala Ala Pro Thr Val Ser Ile Phe Pro Pro Ser Ser
        115                 120                 125

Glu Gln Leu Thr Ser Gly Gly Ala Ser Val Val Cys Phe Leu Asn Asn
    130                 135                 140

Phe Tyr Pro Lys Asp Ile Asn Val Lys Trp Lys Ile Asp Gly Ser Glu
145                 150                 155                 160

Arg Gln Asn Gly Val Leu Asn Ser Trp Thr Asp Gln Asp Ser Lys Asp

165                 170                 175

Ser Thr Tyr Ser Met Ser Ser Thr Leu Thr Leu Thr Lys Asp Glu Tyr
            180                 185                 190

Glu Arg His Asn Ser Tyr Thr Cys Glu Ala Thr His Lys Thr Ser Thr
        195                 200                 205

Ser Pro Ile Val Lys Ser Phe Asn Arg Asn Glu Cys
    210                 215                 220

<210>  3
<211>  437
<212>  PRT
<213>  Artificial sequence

<220>
<223>  An artificially synthesized sequence

<400>  3

Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Gly Pro Thr Pro Asp Thr Ala Tyr Ser Glu Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser
            180                 185                 190

Glu Thr Val Thr Cys Asn Val Ala His Pro Ala Ser Ser Thr Lys Val
        195                 200                 205

Asp Lys Lys Ile Val Pro Arg Asp Cys Gly Cys Lys Pro Cys Ile Cys
    210                 215                 220

Thr Val Lys Glu Val Ser Lys Val Phe Ile Phe Pro Pro Lys Pro Lys
225                 230                 235                 240

Asp Val Leu Thr Ile Thr Leu Thr Pro Lys Val Thr Cys Val Val Val
                245                 250                 255

Asp Ile Ser Lys Asp Asp Pro Glu Val Gln Phe Ser Trp Phe Val Asp
            260                 265                 270

Asp Val Glu Val His Thr Ala Gln Thr Gln Pro Arg Glu Glu Gln Phe
        275                 280                 285

Asn Ser Thr Phe Arg Ser Val Ser Glu Leu Pro Ile Met His Gln Asp
    290                 295                 300

Trp Leu Asn Gly Lys Glu Phe Lys Cys Arg Val Asn Ser Ala Ala Phe
305                 310                 315                 320

Pro Ala Pro Ile Glu Lys Thr Ile Ser Lys Thr Lys Gly Arg Pro Lys
                325                 330                 335

Ala Pro Gln Val Tyr Thr Ile Pro Pro Pro Lys Glu Gln Met Ala Lys
            340                 345                 350

Asp Lys Val Ser Leu Thr Cys Met Ile Thr Asp Phe Phe Pro Glu Asp
        355                 360                 365

Ile Thr Val Glu Trp Gln Trp Asn Gly Gln Pro Ala Glu Asn Tyr Lys
    370                 375                 380

Asn Thr Gln Pro Ile Met Asp Thr Asp Gly Ser Tyr Phe Val Tyr Ser
385                 390                 395                 400

Glu Leu Asn Val Gln Lys Ser Asn Trp Glu Ala Gly Asn Thr Phe Thr

                405                 410                 415

Cys Ser Val Leu His Glu Gly Leu His Asn His His Thr Glu Lys Ser
            420                 425                 430

Leu Ser His Ser Pro
        435

<210> 4
<211> 219
<212> PRT
<213> Artificial sequence

<220>
<223> An artificially synthesized sequence

<400> 4

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Pro Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Gly
                85                  90                  95

Thr Gln Val Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  5
<211>  438
<212>  PRT
<213>  Artificial sequence

<220>
<223>  An artificially synthesized sequence

<400>  5

Gln Val Gln Leu Gln Gln Ser Gly Pro Gln Leu Val Arg Pro Gly Ala
1               5                   10                  15

Ser Val Lys Ile Ser Cys Lys Ala Ser Gly Tyr Ser Phe Thr Ser Tyr
            20                  25                  30

Trp Met His Trp Val Asn Gln Arg Pro Gly Gln Gly Leu Glu Trp Ile
        35                  40                  45

Gly Met Ile Asp Pro Ser Tyr Ser Glu Thr Arg Leu Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Leu Tyr Gly Asn Tyr Phe Asp Tyr Trp Gly Gln Gly Thr Thr Leu
            100                 105                 110

Thr Val Ser Ser Ala Lys Thr Thr Pro Pro Ser Val Tyr Pro Leu Ala
        115                 120                 125

Pro Gly Ser Ala Ala Gln Thr Asn Ser Met Val Thr Leu Gly Cys Leu
    130                 135                 140

Val Lys Gly Tyr Phe Pro Glu Pro Val Thr Val Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Pro Ser Ser Thr Trp Pro
            180                 185                 190

Ser Glu Thr Val Thr Cys Asn Val Ala His Pro Ala Ser Ser Thr Lys
        195                 200                 205

Val Asp Lys Lys Ile Val Pro Arg Asp Cys Gly Cys Lys Pro Cys Ile
    210                 215                 220

Cys Thr Val Lys Glu Val Ser Lys Val Phe Ile Phe Pro Pro Lys Pro
225                 230                 235                 240

Lys Asp Val Leu Thr Ile Thr Leu Thr Pro Lys Val Thr Cys Val Val
                245                 250                 255

Val Asp Ile Ser Lys Asp Asp Pro Glu Val Gln Phe Ser Trp Phe Val
            260                 265                 270

Asp Asp Val Glu Val His Thr Ala Gln Thr Gln Pro Arg Glu Glu Gln
        275                 280                 285

Phe Asn Ser Thr Phe Arg Ser Val Ser Glu Leu Pro Ile Met His Gln
    290                 295                 300

Asp Trp Leu Asn Gly Lys Glu Phe Lys Cys Arg Val Asn Ser Ala Ala
305                 310                 315                 320

Phe Pro Ala Pro Ile Glu Lys Thr Ile Ser Lys Thr Lys Gly Arg Pro
                325                 330                 335

Lys Ala Pro Gln Val Tyr Thr Ile Pro Pro Pro Lys Glu Gln Met Ala
            340                 345                 350

Lys Asp Lys Val Ser Leu Thr Cys Met Ile Thr Asp Phe Phe Pro Glu
        355                 360                 365

Asp Ile Thr Val Glu Trp Gln Trp Asn Gly Gln Pro Ala Glu Asn Tyr
    370                 375                 380

Lys Asn Thr Gln Pro Ile Met Asp Thr Asp Gly Ser Tyr Phe Val Tyr
385                 390                 395                 400

Ser Glu Leu Asn Val Gln Lys Ser Asn Trp Glu Ala Gly Asn Thr Phe
                405                 410                 415

Thr Cys Ser Val Leu His Glu Gly Leu His Asn His His Thr Glu Lys
            420                 425                 430

Ser Leu Ser His Ser Pro
        435

<210>  6
<211>  214
<212>  PRT
<213>  Artificial sequence

<220>
<223>  An artificially synthesized sequence

<400>  6

Asp Ile Gln Met Thr Gln Ser Ser Ser Ser Phe Ser Val Ser Leu Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Asn Ala Pro Arg Leu Leu Ile
        35                  40                  45

Ser Gly Ala Thr Ser Leu Glu Thr Gly Val Pro Ser Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Val Ala Thr Tyr Tyr Cys Gln Gln Tyr Trp Ser Thr Pro Tyr
                85                  90                  95

Thr Phe Gly Gly Gly Thr Lys Leu Glu Val Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  7
<211>  446
<212>  PRT
<213>  Artificial sequence

<220>
<223>  An artificially synthesized sequence

<400>  7

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Tyr Met Asn Trp Ile Arg Gln Pro Pro Gly Gln Ala Pro Glu Trp Leu
        35                  40                  45

Gly Val Ile Arg Asn Lys Gly Asn Gly Tyr Thr Thr Glu Val Asn Thr
    50                  55                  60

Ser Val Lys Gly Arg Phe Thr Ile Ser Arg Asp Asn Thr Gln Asn Ile
65                  70                  75                  80

Leu Tyr Leu Gln Met Asn Ser Leu Arg Ala Glu Asp Thr Ala Ile Tyr
                85                  90                  95

Tyr Cys Ala Arg Gly Gly Pro Tyr Tyr Tyr Ser Gly Asp Asp Ala Pro
            100                 105                 110

Tyr Trp Gly Gln Gly Val Met Val Thr Val Ser Ser Ala Lys Thr Thr
        115                 120                 125

Pro Pro Ser Val Tyr Pro Leu Ala Pro Gly Ser Ala Ala Gln Thr Asn
    130                 135                 140

Ser Met Val Thr Leu Gly Cys Leu Val Lys Gly Tyr Phe Pro Glu Pro
145                 150                 155                 160

Val Thr Val Thr Trp Asn Ser Gly Ser Leu Ser Ser Gly Val His Thr
                165                 170                 175

Phe Pro Ala Val Leu Gln Ser Asp Leu Tyr Thr Leu Ser Ser Ser Val
            180                 185                 190

Thr Val Pro Ser Ser Thr Trp Pro Ser Glu Thr Val Thr Cys Asn Val
        195                 200                 205

Ala His Pro Ala Ser Ser Thr Lys Val Asp Lys Lys Ile Val Pro Arg
    210                 215                 220

Asp Cys Gly Cys Lys Pro Cys Ile Cys Thr Val Lys Glu Val Ser Lys
225                 230                 235                 240

Val Phe Ile Phe Pro Pro Lys Pro Lys Asp Val Leu Thr Ile Thr Leu
                245                 250                 255

Thr Pro Lys Val Thr Cys Val Val Val Asp Ile Ser Lys Asp Asp Pro
            260                 265                 270

Glu Val Gln Phe Ser Trp Phe Val Asp Asp Val Glu Val His Thr Ala
        275                 280                 285

Gln Thr Gln Pro Arg Glu Glu Gln Phe Asn Ser Thr Phe Arg Ser Val
    290                 295                 300

Ser Glu Leu Pro Ile Met His Gln Asp Trp Leu Asn Gly Lys Glu Phe
305                 310                 315                 320

Lys Cys Arg Val Asn Ser Ala Ala Phe Pro Ala Pro Ile Glu Lys Thr
                325                 330                 335

Ile Ser Lys Thr Lys Gly Arg Pro Lys Ala Pro Gln Val Tyr Thr Ile
            340                 345                 350

Pro Pro Pro Lys Glu Gln Met Ala Lys Asp Lys Val Ser Leu Thr Cys
        355                 360                 365

Met Ile Thr Asp Phe Phe Pro Glu Asp Ile Thr Val Glu Trp Gln Trp
    370                 375                 380

Asn Gly Gln Pro Ala Glu Asn Tyr Lys Asn Thr Gln Pro Ile Met Arg
385                 390                 395                 400

Thr Asp Gly Ser Tyr Phe Val Tyr Ser Lys Leu Asn Val Gln Lys Ser
                405                 410                 415

Asn Trp Glu Ala Gly Asn Thr Phe Thr Cys Ser Val Leu His Glu Gly
            420                 425                 430

Leu His Asn His His Thr Glu Lys Ser Leu Ser His Ser Pro
        435                 440                 445

<210>  8
<211>  214
<212>  PRT
<213>  Artificial sequence

<220>
<223>  An artificially synthesized sequence

<400>  8

Asp Ile Glu Leu Thr Gln Ser Pro Ser Phe Leu Ser Thr Ser Leu Gly
1               5                   10                  15

Asn Ser Ile Thr Ile Thr Cys His Ala Ser Gln Asn Ile Lys Gly Trp
            20                  25                  30

Leu Ala Trp Tyr Gln Gln Lys Ser Gly Asn Ala Pro Gln Leu Leu Ile
        35                  40                  45

Tyr Lys Ala Ser Ser Leu Gln Ser Gly Val Pro Ser Arg Phe Ser Gly
    50                  55                  60

Ser Gly Ser Gly Thr Asp Tyr Ile Phe Thr Ile Ser Asn Leu Gln Pro
65                  70                  75                  80

Glu Asp Ile Ala Thr Tyr Tyr Cys Gln His Tyr Gln Ser Phe Pro Trp
                85                  90                  95

Thr Phe Gly Gly Gly Thr Lys Leu Glu Leu Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  9
<211>  438
<212>  PRT
<213>  Artificial sequence

<220>
<223>  An artificially synthesized sequence

<400>  9

Gln Val Gln Leu Gln Gln Ser Gly Pro Gln Leu Val Arg Pro Gly Ala
1               5                   10                  15

Ser Val Lys Ile Ser Cys Lys Ala Ser Gly Tyr Ser Phe Thr Ser Tyr
            20                  25                  30

Trp Met His Trp Val Asn Gln Arg Pro Gly Gln Gly Leu Glu Trp Ile
        35                  40                  45

Gly Met Ile Asp Pro Ser Tyr Ser Glu Thr Arg Leu Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Leu Tyr Gly Asn Tyr Phe Asp Tyr Trp Gly Gln Gly Thr Thr Leu
            100                 105                 110

Thr Val Ser Ser Ala Lys Thr Thr Pro Pro Ser Val Tyr Pro Leu Ala
        115                 120                 125

Pro Gly Ser Ala Ala Gln Thr Asn Ser Met Val Thr Leu Gly Cys Leu
    130                 135                 140

Val Lys Gly Tyr Phe Pro Glu Pro Val Thr Val Thr Trp Asn Ser Gly
145                 150                 155                 160

Ser Leu Ser Ser Gly Val His Thr Phe Pro Ala Val Leu Gln Ser Asp

                165                 170                 175

Leu Tyr Thr Leu Ser Ser Ser Val Thr Val Pro Ser Ser Thr Trp Pro
            180                 185                 190

Ser Glu Thr Val Thr Cys Asn Val Ala His Pro Ala Ser Ser Thr Lys
        195                 200                 205

Val Asp Lys Lys Ile Val Pro Arg Asp Cys Gly Cys Lys Pro Cys Ile
    210                 215                 220

Cys Thr Val Lys Glu Val Ser Lys Val Phe Ile Phe Pro Pro Lys Pro
225                 230                 235                 240

Lys Asp Val Leu Thr Ile Thr Leu Thr Pro Lys Val Thr Cys Val Val
                245                 250                 255

Val Asp Ile Ser Lys Asp Asp Pro Glu Val Gln Phe Ser Trp Phe Val
            260                 265                 270

Asp Asp Val Glu Val His Thr Ala Gln Thr Gln Pro Arg Glu Glu Gln
        275                 280                 285

Phe Asn Ser Thr Phe Arg Ser Val Ser Glu Leu Pro Ile Met His Gln
    290                 295                 300

Asp Trp Leu Asn Gly Lys Glu Phe Lys Cys Arg Val Asn Ser Ala Ala
305                 310                 315                 320

Phe Pro Ala Pro Ile Glu Lys Thr Ile Ser Lys Thr Lys Gly Arg Pro
                325                 330                 335

Lys Ala Pro Gln Val Tyr Thr Ile Pro Pro Pro Lys Glu Gln Met Ala
            340                 345                 350

Lys Asp Lys Val Ser Leu Thr Cys Met Ile Thr Asp Phe Phe Pro Glu
        355                 360                 365

Asp Ile Thr Val Glu Trp Gln Trp Asn Gly Gln Pro Ala Glu Asn Tyr
    370                 375                 380

Lys Asn Thr Gln Pro Ile Met Arg Thr Asp Gly Ser Tyr Phe Val Tyr
385                 390                 395                 400

Ser Lys Leu Asn Val Gln Lys Ser Asn Trp Glu Ala Gly Asn Thr Phe
                405                 410                 415

Thr Cys Ser Val Leu His Glu Gly Leu His Asn His His Thr Glu Lys
            420                 425                 430

Ser Leu Ser His Ser Pro
        435

<210> 10
<211> 439
<212> PRT
<213> Artificial sequence

<220>
<223> An artificially synthesized sequence

<400> 10

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Ile Arg Gln Pro Pro Gly Gln Gly Leu Glu Trp Ile
        35                  40                  45

Gly Ala Ile Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser

180                 185                 190

Glu Thr Val Thr Cys Asn Val Ala His Pro Ala Ser Ser Thr Lys Val
        195                 200                 205

Asp Lys Lys Ile Val Pro Arg Asp Cys Gly Cys Lys Pro Cys Ile Cys
    210                 215                 220

Thr Val Lys Glu Val Ser Lys Val Phe Ile Phe Pro Pro Lys Pro Lys
225                 230                 235                 240

Asp Val Leu Thr Ile Thr Leu Thr Pro Lys Val Thr Cys Val Val Val
                245                 250                 255

Asp Ile Ser Lys Asp Asp Pro Glu Val Gln Phe Ser Trp Phe Val Asp
            260                 265                 270

Asp Val Glu Val His Thr Ala Gln Thr Gln Pro Arg Glu Glu Gln Phe
        275                 280                 285

Asn Ser Thr Phe Arg Ser Val Ser Glu Leu Pro Ile Met His Gln Asp
    290                 295                 300

Trp Leu Asn Gly Lys Glu Phe Lys Cys Arg Val Asn Ser Ala Ala Phe
305                 310                 315                 320

Pro Ala Pro Ile Glu Lys Thr Ile Ser Lys Thr Lys Gly Arg Pro Lys
                325                 330                 335

Ala Pro Gln Val Tyr Thr Ile Pro Pro Pro Lys Glu Gln Met Ala Lys
            340                 345                 350

Asp Lys Val Ser Leu Thr Cys Met Ile Thr Asp Phe Phe Pro Glu Asp
        355                 360                 365

Ile Thr Val Glu Trp Gln Trp Asn Gly Gln Pro Ala Glu Asn Tyr Lys
    370                 375                 380

Asn Thr Gln Pro Ile Met Asp Thr Asp Gly Ser Tyr Phe Val Tyr Ser
385                 390                 395                 400

Glu Leu Asn Val Gln Lys Ser Asn Trp Glu Ala Gly Asn Thr Phe Thr
                405                 410                 415

Cys Ser Val Leu His Glu Gly Leu His Asn His His Thr Glu Lys Ser
            420                 425                 430

Leu Ser His Ser Pro Gly Lys
        435

<210>  11
<211>  219
<212>  PRT
<213>  Artificial sequence

<220>
<223>  An artificially synthesized sequence

<400>  11

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser

195                 200                 205

Pro Ile Val Lys Ser Phe Asn Arg Asn Glu Cys
    210                 215

<210>  12
<211>  440
<212>  PRT
<213>  Artificial sequence

<220>
<223>  An artificially synthesized sequence

<400>  12

Glu Val Gln Leu Val Glu Ser Gly Gly Gly Leu Val Gln Pro Gly Lys
1               5                   10                  15

Ser Leu Lys Leu Ser Cys Glu Ala Ser Gly Phe Thr Phe Ser Gly Tyr
            20                  25                  30

Gly Met His Trp Val Arg Gln Ala Pro Gly Arg Gly Leu Glu Ser Val
        35                  40                  45

Ala Tyr Ile Thr Ser Ser Ser Ile Asn Ile Lys Tyr Ala Asp Ala Val
    50                  55                  60

Lys Gly Arg Phe Thr Val Ser Arg Asp Asn Ala Lys Asn Leu Leu Phe
65                  70                  75                  80

Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser Ala Lys Thr Thr Pro Pro Ser Val Tyr Pro Leu Ala
        115                 120                 125

Pro Gly Ser Ala Ala Gln Thr Asn Ser Met Val Thr Leu Gly Cys Leu
    130                 135                 140

Val Lys Gly Tyr Phe Pro Glu Pro Val Thr Val Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Pro Ser Ser Thr Trp Pro
            180                 185                 190

Ser Glu Thr Val Thr Cys Asn Val Ala His Pro Ala Ser Ser Thr Lys
        195                 200                 205

Val Asp Lys Lys Ile Val Pro Arg Asp Cys Gly Cys Lys Pro Cys Ile
    210                 215                 220

Cys Thr Val Lys Glu Val Ser Lys Val Phe Ile Phe Pro Pro Lys Pro
225                 230                 235                 240

Lys Asp Val Leu Thr Ile Thr Leu Thr Pro Lys Val Thr Cys Val Val
                245                 250                 255

Val Asp Ile Ser Lys Asp Asp Pro Glu Val Gln Phe Ser Trp Phe Val
            260                 265                 270

Asp Asp Val Glu Val His Thr Ala Gln Thr Gln Pro Arg Glu Glu Gln
        275                 280                 285

Phe Asn Ser Thr Phe Arg Ser Val Ser Glu Leu Pro Ile Met His Gln
    290                 295                 300

Asp Trp Leu Asn Gly Lys Glu Phe Lys Cys Arg Val Asn Ser Ala Ala
305                 310                 315                 320

Phe Pro Ala Pro Ile Glu Lys Thr Ile Ser Lys Thr Lys Gly Arg Pro
                325                 330                 335

Lys Ala Pro Gln Val Tyr Thr Ile Pro Pro Pro Lys Glu Gln Met Ala
            340                 345                 350

Lys Asp Lys Val Ser Leu Thr Cys Met Ile Thr Asp Phe Phe Pro Glu
        355                 360                 365

Asp Ile Thr Val Glu Trp Gln Trp Asn Gly Gln Pro Ala Glu Asn Tyr
    370                 375                 380

Lys Asn Thr Gln Pro Ile Met Arg Thr Asp Gly Ser Tyr Phe Val Tyr
385                 390                 395                 400

Ser Lys Leu Asn Val Gln Lys Ser Asn Trp Glu Ala Gly Asn Thr Phe
                405                 410                 415

Thr Cys Ser Val Leu His Glu Gly Leu His Asn His His Thr Glu Lys
            420                 425                 430

Ser Leu Ser His Ser Pro Gly Lys

435                 440

<210>  13
<211>  214
<212>  PRT
<213>  Artificial sequence

<220>
<223>  An artificially synthesized sequence

<400>  13

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80

Glu Asp Ile Gly Ser Tyr Tyr Cys Gln Gln Tyr Tyr Asn Tyr Pro Trp
                85                  90                  95

Thr Phe Gly Pro Gly Thr Lys Leu Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  14
<211>  5
<212>  PRT
<213>  Artificial sequence

<220>
<223>  An artificially synthesized sequence

<400>  14

Asn Ala Ala Ile Tyr
1               5

<210>  15
<211>  19
<212>  PRT
<213>  Artificial sequence

<220>
<223>  An artificially synthesized sequence

<400>  15

Arg Ile Arg Thr Arg Pro Ser Lys Tyr Ala Thr Asp Tyr Ala Asp Ser
1               5                   10                  15

Val Arg Gly

<210>  16
<211>  9
<212>  PRT
<213>  Artificial sequence

<220>
<223>  An artificially synthesized sequence

<400>  16

Arg Ala Thr Glu Asp Val Pro Phe Tyr
1               5

<210>  17
<211>  120
<212>  PRT
<213>  Artificial sequence

<220>
<223>  An artificially synthesized sequence

<400>  17

Ala Val Gln Ile Val Glu Ser Gly Gly Gly Leu Val Gln Pro Lys Glu

1               5                   10                  15

Ser Leu Lys Ile Ser Cys Thr Ala Ser Gly Phe Thr Phe Ser Asn Ala
            20                  25                  30

Ala Ile Tyr Trp Val Arg Gln Thr Pro Gly Lys Gly Leu Glu Trp Val
        35                  40                  45

Gly Arg Ile Arg Thr Arg Pro Ser Lys Tyr Ala Thr Asp Tyr Ala Asp
    50                  55                  60

Ser Val Arg Gly Arg Phe Thr Ile Ser Arg Asp Asp Ser Lys Ser Met
65                  70                  75                  80

Val Tyr Leu Gln Met Asp Asn Leu Arg Thr Glu Asp Thr Ala Met Tyr
                85                  90                  95

Tyr Cys Thr Pro Arg Ala Thr Glu Asp Val Pro Phe Tyr Trp Gly Gln
            100                 105                 110

Gly Val Met Val Thr Val Ser Ser
        115                 120

<210>  18
<211>  17
<212>  PRT
<213>  Artificial sequence

<220>
<223>  An artificially synthesized sequence

<400>  18

Lys Ser Ser Gln Ser Val Leu Tyr Asp Glu Asn Lys Lys Asn Tyr Leu
1               5                   10                  15

Ala

<210>  19
<211>  7
<212>  PRT
<213>  Artificial sequence

<220>
<223>  An artificially synthesized sequence

<400>  19

Trp Ala Ser Thr Gly Glu Ser
1               5

<210>  20
<211>  9
<212>  PRT
<213>  Artificial sequence

<220>
<223>  An artificially synthesized sequence

<400>  20

Gln Gln Tyr Tyr Asp Phe Pro Pro Thr
1               5


<210>  21
<211>  113
<212>  PRT
<213>  Artificial sequence

<220>
<223>  An artificially synthesized sequence

<400>  21

Asp Ile Val Met Thr Gln Thr Pro Ser Ser Gln Ala Val Ser Ala Gly
1               5                   10                  15


Glu Lys Val Thr Met Asn Cys Lys Ser Ser Gln Ser Val Leu Tyr Asp
            20                  25                  30


Glu Asn Lys Lys Asn Tyr Leu Ala Trp Tyr Gln Gln Lys Ser Gly Gln
        35                  40                  45


Ser Pro Lys Leu Leu Ile Tyr Trp Ala Ser Thr Gly Glu Ser Gly Val
    50                  55                  60


Pro Asp Arg Phe Ile Gly Ser Gly Ser Gly Thr Asp Phe Thr Leu Thr
65                  70                  75                  80


Ile Ser Ser Val Gln Ala Glu Asp Leu Ala Val Tyr Tyr Cys Gln Gln
                85                  90                  95


Tyr Tyr Asp Phe Pro Pro Thr Phe Gly Gly Gly Thr Lys Leu Glu Leu
            100                 105                 110


Lys



<210>  22
<211>  5
<212>  PRT
<213>  Artificial sequence

<220>
<223>  An artificially synthesized sequence

<400>  22

Asp Tyr Glu Met His
1               5


<210>  23
<211>  17
<212>  PRT
<213>  Artificial sequence

<220>
<223>  An artificially synthesized sequence

<400>  23

Ala Ile Asp Gly Pro Thr Pro Asp Thr Ala Tyr Ser Glu Lys Phe Lys
1               5                   10                  15


Gly




<210>  24
<211>  6
<212>  PRT
<213>  Artificial sequence

<220>
<223>  An artificially synthesized sequence

<400>  24

Phe Tyr Ser Tyr Thr Tyr
1               5


<210>  25
<211>  115
<212>  PRT
<213>  Artificial sequence

<220>
<223>  An artificially synthesized sequence

<400>  25

Gln Val Gln Leu Val Gln Ser Gly Ala Glu Val Lys Lys Pro Gly Ala
1               5                   10                  15


Ser Val Thr Val Ser Cys Lys Ala Ser Gly Tyr Thr Phe Thr Asp Tyr
            20                  25                  30


Glu Met His Trp Ile Arg Gln Pro Pro Gly Glu Gly Leu Glu Trp Ile
        35                  40                  45


Gly Ala Ile Asp Gly Pro Thr Pro Asp Thr Ala Tyr Ser Glu Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115

<210>  26
<211>  16
<212>  PRT
<213>  Artificial sequence

<220>
<223>  An artificially synthesized sequence

<400>  26

Arg Ser Ser Gln Pro Leu Val His Ser Asn Arg Asn Thr Tyr Leu His
1               5                   10                  15

<210>  27
<211>  7
<212>  PRT
<213>  Artificial sequence

<220>
<223>  An artificially synthesized sequence

<400>  27

Lys Val Ser Asn Arg Phe Ser
1               5

<210>  28
<211>  9
<212>  PRT
<213>  Artificial sequence

<220>
<223>  An artificially synthesized sequence

<400>  28

Gly Gln Gly Thr Gln Val Pro Tyr Thr
1               5

<210>  29
<211>  112
<212>  PRT

<213>  Artificial sequence

<220>
<223>  An artificially synthesized sequence

<400>  29

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Pro Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Gly
                85                  90                  95

Thr Gln Val Pro Tyr Thr Phe Gly Gln Gly Thr Lys Leu Glu Ile Lys
            100                 105                 110


<210>  30
<211>  5
<212>  PRT
<213>  Artificial sequence

<220>
<223>  An artificially synthesized sequence

<400>  30

Asp Phe Tyr Met Asn
1               5


<210>  31
<211>  19
<212>  PRT
<213>  Artificial sequence

<220>
<223>  An artificially synthesized sequence

<400>  31

Val Ile Arg Asn Lys Gly Asn Gly Tyr Thr Thr Glu Val Asn Thr Ser
1               5                   10                  15

Val Lys Gly

<210> 32
<211> 13
<212> PRT
<213> Artificial sequence

<220>
<223> An artificially synthesized sequence

<400> 32

Gly Gly Pro Tyr Tyr Tyr Ser Gly Asp Asp Ala Pro Tyr
1               5                   10

<210> 33
<211> 124
<212> PRT
<213> Artificial sequence

<220>
<223> An artificially synthesized sequence

<400> 33

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asp Phe
            20                  25                  30

Tyr Met Asn Trp Ile Arg Gln Pro Pro Gly Gln Ala Pro Glu Trp Leu
        35                  40                  45

Gly Val Ile Arg Asn Lys Gly Asn Gly Tyr Thr Thr Glu Val Asn Thr
    50                  55                  60

Ser Val Lys Gly Arg Phe Thr Ile Ser Arg Asp Asn Thr Gln Asn Ile
65                  70                  75                  80

Leu Tyr Leu Gln Met Asn Ser Leu Arg Ala Glu Asp Thr Ala Ile Tyr
                85                  90                  95

Tyr Cys Ala Arg Gly Gly Pro Tyr Tyr Tyr Ser Gly Asp Asp Ala Pro
            100                 105                 110

Tyr Trp Gly Gln Gly Val Met Val Thr Val Ser Ser
        115                 120

<210> 34
<211> 11
<212> PRT

<213>  Artificial sequence

<220>
<223>  An artificially synthesized sequence

<400>  34

His Ala Ser Gln Asn Ile Lys Gly Trp Leu Ala
1               5                   10


<210>  35
<211>  7
<212>  PRT
<213>  Artificial sequence

<220>
<223>  An artificially synthesized sequence

<400>  35

Lys Ala Ser Ser Leu Gln Ser
1               5


<210>  36
<211>  9
<212>  PRT
<213>  Artificial sequence

<220>
<223>  An artificially synthesized sequence

<400>  36

Gln His Tyr Gln Ser Phe Pro Trp Thr
1               5


<210>  37
<211>  107
<212>  PRT
<213>  Artificial sequence

<220>
<223>  An artificially synthesized sequence

<400>  37

Asp Ile Glu Leu Thr Gln Ser Pro Ser Phe Leu Ser Thr Ser Leu Gly
1               5                   10                  15


Asn Ser Ile Thr Ile Thr Cys His Ala Ser Gln Asn Ile Lys Gly Trp
            20                  25                  30


Leu Ala Trp Tyr Gln Gln Lys Ser Gly Asn Ala Pro Gln Leu Leu Ile
        35                  40                  45


Tyr Lys Ala Ser Ser Leu Gln Ser Gly Val Pro Ser Arg Phe Ser Gly
    50                  55                  60

Ser Gly Ser Gly Thr Asp Tyr Ile Phe Thr Ile Ser Asn Leu Gln Pro
65                  70                  75                  80

Glu Asp Ile Ala Thr Tyr Tyr Cys Gln His Tyr Gln Ser Phe Pro Trp
                85                  90                  95

Thr Phe Gly Gly Gly Thr Lys Leu Glu Leu Lys
            100                 105

<210>  38
<211>  5
<212>  PRT
<213>  Artificial sequence

<220>
<223>  An artificially synthesized sequence

<400>  38

Gly Tyr Gly Met His
1               5

<210>  39
<211>  17
<212>  PRT
<213>  Artificial sequence

<220>
<223>  An artificially synthesized sequence

<400>  39

Tyr Ile Thr Ser Ser Ser Ile Asn Ile Lys Tyr Ala Asp Ala Val Lys
1               5                   10                  15

Gly

<210>  40
<211>  7
<212>  PRT
<213>  Artificial sequence

<220>
<223>  An artificially synthesized sequence

<400>  40

Phe Asp Trp Asp Lys Asn Tyr
1               5

<210>  41
<211>  116
<212>  PRT

<213>  Artificial sequence

<220>
<223>  An artificially synthesized sequence

<400>  41

Glu Val Gln Leu Val Glu Ser Gly Gly Gly Leu Val Gln Pro Gly Lys
1               5                   10                  15

Ser Leu Lys Leu Ser Cys Glu Ala Ser Gly Phe Thr Phe Ser Gly Tyr
            20                  25                  30

Gly Met His Trp Val Arg Gln Ala Pro Gly Arg Gly Leu Glu Ser Val
        35                  40                  45

Ala Tyr Ile Thr Ser Ser Ser Ile Asn Ile Lys Tyr Ala Asp Ala Val
    50                  55                  60

Lys Gly Arg Phe Thr Val Ser Arg Asp Asn Ala Lys Asn Leu Leu Phe
65                  70                  75                  80

Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser
        115

<210>  42
<211>  11
<212>  PRT
<213>  Artificial sequence

<220>
<223>  An artificially synthesized sequence

<400>  42

Gln Ala Ser Gln Asp Ile Ser Asn Tyr Leu Asn
1               5                   10

<210>  43
<211>  7
<212>  PRT
<213>  Artificial sequence

<220>
<223>  An artificially synthesized sequence

<400>  43

Tyr Thr Asn Lys Leu Ala Asp
1               5

<210>  44
<211>  9
<212>  PRT
<213>  Artificial sequence

<220>
<223>  An artificially synthesized sequence

<400>  44

Gln Gln Tyr Tyr Asn Tyr Pro Trp Thr
1               5

<210>  45
<211>  107
<212>  PRT
<213>  Artificial sequence

<220>
<223>  An artificially synthesized sequence

<400>  45

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80

Glu Asp Ile Gly Ser Tyr Tyr Cys Gln Gln Tyr Tyr Asn Tyr Pro Trp
                85                  90                  95

Thr Phe Gly Pro Gly Thr Lys Leu Glu Ile Lys
            100                 105

<210>  46
<211>  5
<212>  PRT
<213>  Artificial sequence

<220>
<223>  An artificially synthesized sequence

<400>  46

Asp Tyr Glu Met His
1               5


<210>  47
<211>  17
<212>  PRT
<213>  Artificial sequence

<220>
<223>  An artificially synthesized sequence

<400>  47

Ala Ile Asp Pro Lys Thr Gly Asp Thr Ala Tyr Ser Gln Lys Phe Lys
1               5                   10                  15


Gly




<210>  48
<211>  6
<212>  PRT
<213>  Artificial sequence

<220>
<223>  An artificially synthesized sequence

<400>  48

Phe Tyr Ser Tyr Thr Tyr
1               5


<210>  49
<211>  115
<212>  PRT
<213>  Artificial sequence

<220>
<223>  An artificially synthesized sequence

<400>  49

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Glu Met His Trp Ile Arg Gln Pro Pro Gly Gln Gly Leu Glu Trp Ile
        35                  40                  45


Gly Ala Ile Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115

<210>  50
<211>  16
<212>  PRT
<213>  Artificial sequence

<220>
<223>  An artificially synthesized sequence

<400>  50

Arg Ser Ser Gln Ser Leu Val His Ser Asn Arg Asn Thr Tyr Leu His
1               5                   10                  15

<210>  51
<211>  7
<212>  PRT
<213>  Artificial sequence

<220>
<223>  An artificially synthesized sequence

<400>  51

Lys Val Ser Asn Arg Phe Ser
1               5

<210>  52
<211>  9
<212>  PRT
<213>  Artificial sequence

<220>
<223>  An artificially synthesized sequence

<400>  52

Ser Gln Asn Thr His Val Pro Pro Thr
1               5

<210>  53
<211>  112
<212>  PRT

<213>  Artificial sequence

<220>
<223>  An artificially synthesized sequence

<400>  53

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210>  54
<211>  437
<212>  PRT
<213>  Artificial sequence

<220>
<223>  An artificially synthesized sequence

<400>  54

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Leu Leu Trp Asn Tyr Trp Gly Gln Gly Thr Leu Val Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser
            180                 185                 190

Glu Thr Val Thr Cys Asn Val Ala His Pro Ala Ser Ser Thr Lys Val
        195                 200                 205

Asp Lys Lys Ile Val Pro Arg Asp Cys Gly Cys Lys Pro Cys Ile Cys
    210                 215                 220

Thr Val Lys Glu Val Ser Lys Val Phe Ile Phe Pro Pro Lys Pro Lys
225                 230                 235                 240

Asp Val Leu Thr Ile Thr Leu Thr Pro Lys Val Thr Cys Val Val Val
                245                 250                 255

Asp Ile Ser Lys Asp Asp Pro Glu Val Gln Phe Ser Trp Phe Val Asp
            260                 265                 270

Asp Val Glu Val His Thr Ala Gln Thr Gln Pro Arg Glu Glu Gln Phe
        275                 280                 285

Asn Ser Thr Phe Arg Ser Val Ser Glu Leu Pro Ile Met His Gln Asp
    290                 295                 300

Trp Leu Asn Gly Lys Glu Phe Lys Cys Arg Val Asn Ser Ala Ala Phe
305                 310                 315                 320

Pro Ala Pro Ile Glu Lys Thr Ile Ser Lys Thr Lys Gly Arg Pro Lys

325                 330                 335

Ala Pro Gln Val Tyr Thr Ile Pro Pro Pro Lys Glu Gln Met Ala Lys
            340                 345                 350

Asp Lys Val Ser Leu Thr Cys Met Ile Thr Asp Phe Phe Pro Glu Asp
        355                 360                 365

Ile Thr Val Glu Trp Gln Trp Asn Gly Gln Pro Ala Glu Asn Tyr Lys
    370                 375                 380

Asn Thr Gln Pro Ile Met Asp Thr Asp Gly Ser Tyr Phe Val Tyr Ser
385                 390                 395                 400

Glu Leu Asn Val Gln Lys Ser Asn Trp Glu Ala Gly Asn Thr Phe Thr
                405                 410                 415

Cys Ser Val Leu His Glu Gly Leu His Asn His His Thr Glu Lys Ser
            420                 425                 430

Leu Ser His Ser Pro
        435

<210>  55
<211>  216
<212>  PRT
<213>  Artificial sequence

<220>
<223>  An artificially synthesized sequence

<400>  55

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Ile Ile
        35                  40                  45

Tyr Gly Ala Ser Thr Thr Ala Ser Gly Ile Pro Ala Arg Phe Ser Ala
    50                  55                  60

Ser Gly Ser Gly Thr Asp Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Pro
                85                  90                  95

Ala Tyr Thr Phe Gly Gln Gly Thr Lys Leu Glu Ile Lys Arg Thr Val
            100                 105                 110

Ala Ala Pro Ser Val Phe Ile Phe Pro Pro Ser Asp Glu Gln Leu Lys
        115                 120                 125

Ser Gly Thr Ala Ser Val Val Cys Leu Leu Asn Asn Phe Tyr Pro Arg
    130                 135                 140

Glu Ala Lys Val Gln Trp Lys Val Asp Asn Ala Leu Gln Ser Gly Asn
145                 150                 155                 160

Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190

Val Tyr Ala Cys Glu Val Thr His Gln Gly Leu Ser Ser Pro Val Thr
        195                 200                 205

Lys Ser Phe Asn Arg Gly Glu Cys
    210                 215

<210>  56
<211>  5
<212>  PRT
<213>  Artificial sequence

<220>
<223>  An artificially synthesized sequence

<400>  56

Ser Tyr Ala Ile Ser
1               5

<210>  57
<211>  17
<212>  PRT
<213>  Artificial sequence

<220>
<223>  An artificially synthesized sequence

<400>  57

Gly Ile Ile Pro Ile Phe Gly Thr Ala Asn Tyr Ala Gln Lys Phe Gln
1               5                   10                  15

Gly

<210>  58
<211>  6
<212>  PRT
<213>  Artificial sequence

<220>
<223>  An artificially synthesized sequence

<400>  58

Gly Leu Leu Trp Asn Tyr
1               5


<210>  59
<211>  115
<212>  PRT
<213>  Artificial sequence

<220>
<223>  An artificially synthesized sequence

<400>  59

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Leu Leu Trp Asn Tyr Trp Gly Gln Gly Thr Leu Val Thr
            100                 105                 110

Val Ser Ser
        115


<210>  60
<211>  11
<212>  PRT
<213>  Artificial sequence

<220>
<223>  An artificially synthesized sequence

<400>  60

Arg Ala Ser Gln Ser Val Ser Ser Asn Leu Ala
1               5                   10


<210>  61
<211>  7
<212>  PRT
<213>  Artificial sequence

<220>
<223>  An artificially synthesized sequence

<400>  61

Gly Ala Ser Thr Thr Ala Ser
1               5


<210>  62
<211>  11
<212>  PRT
<213>  Artificial sequence

<220>
<223>  An artificially synthesized sequence

<400>  62

Gln Gln Tyr Asn Asn Trp Pro Pro Ala Tyr Thr
1               5                   10


<210>  63
<211>  109
<212>  PRT
<213>  Artificial sequence

<220>
<223>  An artificially synthesized sequence

<400>  63

Glu Ile Val Met Thr Gln Ser Pro Ala Thr Leu Ser Val Ser Pro Gly
1               5                   10                  15


Glu Arg Ala Thr Leu Ser Cys Arg Ala Ser Gln Ser Val Ser Ser Asn
            20                  25                  30


Leu Ala Trp Tyr Gln Gln Lys Pro Gly Gln Ala Pro Arg Leu Ile Ile
        35                  40                  45


Tyr Gly Ala Ser Thr Thr Ala Ser Gly Ile Pro Ala Arg Phe Ser Ala
    50                  55                  60

Ser Gly Ser Gly Thr Asp Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Pro
                85                  90                  95

Ala Tyr Thr Phe Gly Gln Gly Thr Lys Leu Glu Ile Lys
            100                 105

## Description

### [Technical Field]

In one embodiment, the present invention relates to multispecific antigen-binding molecules capable of inducing phagocytosis of target cells by antigen-presenting cells through crosslinking antigen-presenting cells to target cells. For example, the present invention relates to bispecific antibodies capable of inducing phagocytosis of target cells by antigen-presenting cells through crosslinking antigen-presenting cells to target cells.

The present invention also relates to compositions for inducing an antitumor immune response and compositions for treating or preventing cancer, which comprise such multispecific antigen-binding molecules. The present invention further relates to methods for treating or preventing cancer, comprising the step of administering such multispecific antigen binding molecules.

### [Background Art]

Antibodies are proteins which specifically bind to an antigen with high affinity. It is known that various molecules ranging from low-molecular compounds to proteins can be antigens. Since the technique for producing monoclonal antibodies was developed, antibody modification techniques have advanced, making it easy to obtain antibodies that recognize a particular molecule.

Antibodies are drawing attention as pharmaceuticals because they are highly stable in blood plasma and have less side effects. Not only do antibodies bind to an antigen and exhibit agonistic or antagonistic effects, but they also induce cytotoxic activity mediated by effector cells (also referred to as effector functions) including ADCC (Antibody Dependent Cytotoxicity), ADCP (Antibody Dependent Cell Phagocytosis), and CDC (Complement Dependent Cytotoxicity). Taking advantage of these antibody functions, pharmaceuticals for cancer, immune diseases, chronic disease, infections, etc. have been developed (Nat Rev Drug Discov. 2018 Mar;17(3):197-223 (NPL 1)).

For example, cancer immunotherapy has received a great deal of attention in recent years in cancer treatment, and this is especially so for checkpoint inhibitor antibodies targeting PD-1/PD-L1 and CTLA-4 that are widely used clinically (NPL 2, NPL 3, and NPL 4). Checkpoint inhibitors elicit antitumor effects by activating immune cells. However, problems with checkpoint inhibitors include the risk of side effects due to activation of autoreactive immune cells and the limited number of patients in which they are effective. Therefore, there is a strong demand for the development of agents having fewer side effects which are capable of eliciting an antitumor effect of immune cells, or agents capable of efficiently increasing the effect of checkpoint inhibitors.

Antigen-presenting cells activate antigen-reactive T cells by processing phagocytosed antigens and then presenting them on their major histocompatibility complexes (MHCs). Antigen-reactive T cells are considered to be important for the efficacy of checkpoint inhibitors (NPL 5).

Dendritic cells are a type of typical antigen-presenting cells and can efficiently activate antigen-reactive T cells. Therefore, they are considered to play a very important role also in cancer immunotherapy (NPL 6).

As one of the cancer immunotherapies targeting dendritic cells, there is a method in which dendritic cells cultured *ex vivo* are allowed to phagocytose a cancer antigen and then transferred into the body to activate tumor-reactive T cells. This has already been approved as a treatment for prostate cancer (NPL 7). However, in this method, there are many conditions that have to be optimized to maximize the original function of dendritic cells, such as culture conditions and activation conditions of artificially induced dendritic cells, routes for transferring dendritic cells, etc., and thus, sufficient therapeutic effects still have not been obtained. Therefore, in recent years, as another endeavor, there is an attempt, which is being clinically evaluated, to conjugate a cancer antigen with an antibody that recognizes an antigen on dendritic cells and allow dendritic cells that naturally exist in the body to phagocytose these conjugates (NPL 8). However, it is suggested that reactive T cells activated by this method are only T cells corresponding to the antigen conjugated to the antibody, and that T cells that react to other cancer antigens cannot be activated (NPL 9).

### [Citation List]

### [Non-Patent Literature]

[NPL 1] Carter PJ, Lazar GA. Nat Rev Drug Discov. 2018 Mar; 17(3): 197-223.
[NPL 2] Safety, Activity, and Immune Correlates of Anti-PD-1 Antibody in Cancer. N Engl J Med 2012; 366:2443-2454
[NPL 3] Atezolizumab in patients with locally advanced and metastatic urothelial carcinoma who have progressed following treatment with platinum-based chemotherapy: a single-arm, multicentre, phase 2 trial. The Lancet Volume 387, Issue 10031, 7-13 May 2016, Pages 1909-1920
[NPL 4] Improved Survival with Ipilimumab in Patients with Metastatic Melanoma. N Engl J Med 2010; 363:711-723
[NPL 5] PD-1 blockade induces responses by inhibiting adaptive immune resistance. Nature volume 515, pages 568-571 (27 November 2014)
[NPL 6] Cancer immunotherapy via dendritic cells. Nature Reviews Cancer volume 12, pages 265-277 (2012)
[NPL 7] Sipuleucel-T Immunotherapy for Castration-Resistant Prostate Cancer. July 29, 2010, N Engl J Med 2010; 363:411-422
[NPL 8] Induction of Antigen-Specific Immunity with a Vaccine Targeting NY-ESO-1 to the Dendritic Cell Receptor DEC-205. Science Translational Medicine 16 Apr 2014:Vol. 6, Issue 232, pp. 232ra51 Pages 385-392
[NPL 9] A Phase II Randomized Study of CDX-1401, a Dendritic Cell Targeting NY-ESO-1 Vaccine, in Patients with Malignant Melanoma Pre-Treated with Recombinant CDX-301, a Recombinant Human Flt3 Ligand. 2016 ASCO Annual Meeting, Abstract #:9589

### [Summary of Invention]

### [Technical Problem]

In one embodiment, the present invention has been made in view of the above circumstances, and an objective thereof is to provide a novel cancer immunotherapy using antigen-presenting cells such as dendritic cells.

### [Solution to Problem]

As a result of dedicated research to achieve the above objective, the present inventors crosslinked antigen-presenting cells to target cells using a multispecific antigen-binding molecule that recognizes an antigen on antigen-presenting cells and an antigen on target cells, and discovered that phagocytosis of target cells by antigen-presenting cells can be enhanced.

The present invention is based on such findings, and as examples, provides the following embodiments:
[1] a multispecific antigen-binding molecule comprising a first antigen-binding domain that binds to a first antigen on an antigen-presenting cell and a second antigen-binding domain that binds to a second antigen on a target cell;
[2] the multispecific antigen-binding molecule of [1], wherein said target cell is a cancer cell;
   [2-1] the multispecific antigen-binding molecule of [1], wherein the target cell is a bacterium;
   [2-2] the multispecific antigen-binding molecule of [1], wherein the target cell is a cell infected with a virus or the like;
[3] the multispecific antigen binding molecule of [1] or [2], which can induce phagocytosis of said target cell by said antigen-presenting cell through the crosslinking of said antigen-presenting cell and said target cell via said multispecific antigen-binding molecule;
   [3-1] the multispecific antigen-binding molecule of [3], wherein said target cell is an undisrupted target cell;
[4] the multispecific antigen-binding molecule of any one of [1] to [3], wherein multiple types of antigen peptides derived from said target cell which has been incorporated into the antigen-presenting cell are presented by the antigen-presenting cell;
[5] the multispecific antigen-binding molecule of any one of [1] to [4], wherein said antigen peptide derived from the target cell and presented on the MHC class I protein of the antigen-presenting cell is derived from an antigen different from the second antigen on the target cell, or from the second antigen on the target cell;
[6] the multispecific antigen-binding molecule of any one of [1] to [5], wherein the antigen-presenting cell is a dendritic cell;
[7] the multispecific antigen-binding molecule of any one of [1] to [6], which can induce cross-presentation by the dendritic cell;
[8] the multispecific antigen-binding molecule of any one of [1] to [7], wherein said first antigen on the antigen-presenting cell is a dendritic cell surface antigen and is capable of inducing dendritic cell cross-presentation;
[9] the multispecific antigen-binding molecule of any one of [1] to [8], wherein said antigen on the surface of the antigen-presenting cell is a C-type lectin receptor or an integrin receptor;
[10] the multispecific antigen-binding molecule of any one of [1] to [9], wherein said first antigen on the antigen-presenting cell is an antigen selected from the group consisting of CLEC9A, DEC205, and CD207;
[11] the multispecific antigen-binding molecule of any one of [1] to [10], wherein the second antigen on the target cell is an antigen selected from the group consisting of GPC3, IL6R, and EpCAM;
[12] the multispecific antigen-binding molecule of any one of [1] to [11], wherein the antigen-binding molecule is an antibody;
[13] the multispecific antigen-binding molecule of any one of [1] to [12], wherein the first antigen-binding domain comprises:
   (1) a heavy chain variable region comprising the CDR1 sequence set forth in SEQ ID NO: 14, the CDR2 sequence set forth in SEQ ID NO: 15 and the CDR3 sequence set forth in SEQ ID NO: 16, and a light chain variable region comprising the CDR1 sequence set forth in SEQ ID NO: 18, the CDR2 sequence set forth in SEQ ID NO: 19, and CDR3 sequence set forth in SEQ ID NO: 20;
   (2) a heavy chain variable region having the amino acid sequence set forth in SEQ ID NO: 17 and a light chain variable region having the amino acid sequence set forth in SEQ ID NO:21; or
   (3) a heavy chain having the amino acid sequence set forth in SEQ ID NO: 1 and a light chain having the amino acid sequence set forth in SEQ ID NO: 2,
   and wherein the second antigen-binding domain comprises:
   (4) a heavy chain variable region comprising the CDR1 sequence set forth in SEQ ID NO: 22, the CDR2 sequence set forth in SEQ ID NO: 23, and the CDR3 sequence set forth in SEQ ID NO: 24, and a light chain variable region comprising the CDR1 sequence set forth in SEQ ID NO:26, the CDR2 sequence set forth in SEQ ID NO: 27, and the CDR3 sequence set forth in SEQ ID NO: 28;
   (5) a heavy chain variable region having the amino acid sequence set forth in SEQ ID NO: 25 and a light chain variable region having the amino acid sequence set forth in SEQ ID NO: 29; or
   (6) a heavy chain having the amino acid sequence set forth in SEQ ID NO: 3 and a light chain having the amino acid sequence set forth in SEQ ID NO: 4;
      [13-1] the multispecific antigen-binding molecule of any one of [1] to [12], wherein the first antigen-binding domain comprises:
      (1) a heavy chain variable region comprising the CDR1 sequence set forth in SEQ ID NO: 14, the CDR2 sequence set forth in SEQ ID NO: 15 and the CDR3 sequence set forth in SEQ ID NO: 16, and a light chain variable region comprising the CDR1 sequence set forth in SEQ ID NO: 18, the CDR2 sequence set forth in SEQ ID NO: 19 and the CDR3 sequence set forth in SEQ ID NO: 20;
      (2) a heavy chain variable region having the amino acid sequence set forth in SEQ ID NO: 17 and a light chain variable region having the amino acid sequence set forth in SEQ ID NO: 21; or
      (3) a heavy chain having the amino acid sequence set forth in SEQ ID NO: 1 and a light chain having the amino acid sequence set forth in SEQ ID NO: 2,
      and wherein the second antigen-binding domain comprises:
      (4) a heavy chain variable region comprising the CDR1 sequence set forth in SEQ ID NO: 56, the CDR2 sequence set forth in SEQ ID NO: 57, and the CDR3 sequence set forth in SEQ ID NO: 58, and a light chain variable region comprising the CDR1 sequence set forth in SEQ ID NO: 60, the CDR2 sequence set forth in SEQ ID NO: 61, and the CDR3 sequence set forth in SEQ ID NO: 63;
      (5) a heavy chain variable region having the amino acid sequence set forth in SEQ ID NO: 59 and a light chain variable region having the amino acid sequence set forth in SEQ ID NO: 63; or
      (6) a heavy chain having the amino acid sequence set forth in SEQ ID NO: 54 and a light chain having the amino acid sequence set forth in SEQ ID NO: 55.
[14] the multispecific antigen-binding molecule of any one of [1] to [12], wherein the first antigen-binding domain comprises:
   (1) a heavy chain variable region comprising the CDR1 sequence set forth in SEQ ID NO: 30, the CDR2 sequence set forth in SEQ ID NO: 31, and the CDR3 sequence set forth in SEQ ID NO: 32; and a light chain variable region comprising the CDR1 sequence set forth in SEQ ID NO: 34, the CDR2 sequence set forth in SEQ ID NO: 35 and the CDR3 sequence set forth in SEQ ID NO: 36;
   (2) a heavy chain variable region having the amino acid sequence set forth in SEQ ID NO: 33 and a light chain variable region having the amino acid sequence set forth in SEQ ID NO: 37; or
   (3) a heavy chain having the amino acid sequence set forth in SEQ ID NO: 7 and a light chain having the amino acid sequence set forth in SEQ ID NO: 8;
   and wherein the second antigen-binding domain comprises:
   (4) a heavy chain variable region comprising the CDR1 sequence set forth in SEQ ID NO: 22, the CDR2 sequence set forth in SEQ ID NO: 23, and the CDR3 sequence set forth in SEQ ID NO: 24, and a light chain variable region comprising the CDR1 sequence set forth in SEQ ID NO: 26, the CDR2 sequence shown in SEQ ID NO: 27 and the CDR3 sequence set forth in SEQ ID NO: 28;
   (5) a heavy chain variable region having the amino acid sequence set forth in SEQ ID NO: 25 and a light chain variable region having the amino acid sequence set forth in SEQ ID NO: 29; or
   (6) a heavy chain having the amino acid sequence set forth in SEQ ID NO: 3 and a light chain having the amino acid sequence set forth in SEQ ID NO: 4;
[15] the multispecific antigen-binding molecule of any one of [1] to [14], wherein the multispecific antigen-binding molecule is used in combination with an activator of antigen-presenting cells, or is conjugated with an activator of antigen-presenting cells;
[16] the multispecific antigen-binding molecule of [10], wherein the conjugated activator is conjugated to the Fc region or Fab region of the multispecific antigen-binding molecule;
[17] the multispecific antigen-binding molecule of [15] or [16], wherein the activator of antigen presenting cells is a dendritic cell activator or a T cell activator;
[18] the multispecific antigen-binding molecule of [15] or [16], wherein the dendritic cell activator is interferon α (IFNa), poly I:C (poly-I:C), or a T cell activator;
[19] a composition for inducing an antitumor immune response, wherein the composition comprises the multispecific antigen-binding molecule of any one of [1] to [18];
[20] the composition of [19], wherein the induction of the antitumor immune response is activation of cytotoxic T cells (CTLs);
[21] a pharmaceutical composition comprising the multispecific antigen-binding molecule of any one of [1] to [18];
[22] a composition for treating or preventing cancer, which comprises the multispecific antigen-binding molecule of any one of [1] to [18]; and
[23] a composition comprising the multispecific antigen-binding molecule of any one of [1] to [18] or the composition of any one of [19] to [22], wherein the composition is for use in combination with an activator of antigen-presenting cells simultaneously or separately.

The present invention also provides the following as other examples:
[24] a method for allowing an MHC class I protein of an antigen-presenting cell to present an antigen peptide derived from a target cell, wherein the method comprises targeting the antigen-presenting cell to the target cell using the multispecific antigen-binding molecule of any one of [1] to [18];
[25] a method for allowing an antigen-presenting cell to present multiple types of antigens derived from a target cell, wherein the method comprises targeting the antigen-presenting cell to the target cell using the multispecific antigen-binding molecule of any one of [1] to [18];
[26] a method of screening for an antigen peptide derived from cancer, wherein the method comprises targeting an antigen-presenting cell to a cancer cell using the multispecific antigen-binding molecule of any one of [1] to [18] and allowing an MHC class I protein of the antigen-presenting cell to present an antigen peptide derived from the cancer cell;
[27] the method of [26], wherein the cancer cell is derived from a human cancer patient;
[28] a peptide obtained by the screening method of [26] or [27];
[29] use of the peptide of [28] for inducing cytotoxic T cells specific to an MHC/peptide complex comprising the peptide of [28];
[30] an isolated nucleic acid encoding an amino acid sequence of the multispecific antigen-binding molecule of any one of [1] to [18];
[31] a host cell comprising the nucleic acid of [30];
[32] a method for producing a multispecific antigen-binding molecule, wherein the method comprises culturing the host cell of [31] so that the multispecific antigen-binding molecule is produced;
[33] the method of [32], wherein the method further comprises recovering the multispecific antigen-binding molecule from the host cell;
[34] a pharmaceutical preparation comprising the multispecific antigen-binding molecule of any one of [1] to [18] and a pharmaceutically acceptable carrier;
[35] the multispecific antigen-binding molecule of any one of [1] to [18], for use as a medicament;
[36] the multispecific antigen-binding molecule of any one of [1] to [18], for use in the treatment or prevention of cancer;
[37] the multispecific antigen-binding molecule of any one of [1] to [18], for use in activating cytotoxic T cells;
[38] use of the multispecific antigen-binding molecule of any one of [1] to [18] in the manufacture of a medicament for treating or preventing cancer;
[39] use of the multispecific antigen-binding molecule of any one of [1] to [18] in the manufacture of a medicament for activating cytotoxic T cells;
[40] a method for treating or preventing cancer, wherein the method comprises administering to an individual an effective amount of the multispecific antigen-binding molecule of any one of [1] to [19]; and
[41] a method for activating cytotoxic T cells in an individual, wherein the method comprises administering to the individual an effective amount of the multispecific antigen-binding molecule of any one of [1] to [18].

Further, the present invention also provides the following as other examples:
[42] a composition for inducing an antitumor immune response (an antitumor immune response-inducing agent), a composition for activating cytotoxic T cells (a cytotoxic T cell activator), a composition for inducing cytotoxicity (a cytotoxicity inducer), a composition for suppressing cell proliferation (a cell proliferation suppressor), a composition for activating immunity against cancer cells or tumor tissue containing cancer cells (an immune activator against cancer cells or tumor tissue containing cancer cells), a composition for preventing or treating cancer (a cancer preventive or therapeutic agent), a composition for treating an individual having cancer (a therapeutic agent for an individual having cancer), or a composition for inducing the presentation of an antigen peptide derived from a cancer cell on an MHC class I protein of an antigen presenting cell (an inducer of the presentation of an antigen peptide derived from a cancer cell on an MHC class I protein of an antigen presenting cell), wherein the composition comprises the multispecific antigen-binding molecule of any one of [1] to [18] as an active ingredient;
[43] use of the multispecific antigen-binding molecule of any one of [1] to [18] in the manufacture of a composition for inducing an antitumor immune response (an antitumor immune response-inducing agent), a composition for activating cytotoxic T cells (a cytotoxic T cell activator), a composition for inducing cytotoxicity (a cytotoxicity inducer), a composition for suppressing cell proliferation (a cell proliferation suppressor), a composition for activating immunity against cancer cells or tumor tissue containing cancer cells (an immune activator against cancer cells or tumor tissue containing cancer cells), a composition for preventing or treating cancer (a cancer preventive or therapeutic agent), a composition for treating an individual having cancer (a therapeutic agent for an individual having cancer), or a composition for inducing the presentation of an antigen peptide derived from a cancer cell on an MHC class I protein of an antigen presenting cell (an inducer of the presentation of an antigen peptide derived from a cancer cell on an MHC class I protein of an antigen presenting cell);
[44] the multispecific antigen-binding molecule of [1] to [18] for use in the induction of an antitumor immune response, activation of cytotoxic T cells, induction of cytotoxicity, suppression of cell proliferation, activation of immunity against cancer cells or to tumor tissue containing cancer cells, prevention or treatment of cancer, treatment of an individual having cancer, or induction of presentation of an antigen peptide derived from a cancer cell on an MHC class I protein of an antigen-presenting cell; and
[45] a method for inducing an antitumor immune response, a method for activating cytotoxic T cells, a method for inducing cytotoxicity, a method for suppressing cell proliferation, a method for activating immunity against cancer cells or tumor tissue containing cancer cells, a method for preventing or treating cancer, a method for treating an individual having cancer, or a method for allowing an MHC class I protein of an antigen-presenting cell to present an antigen peptide derived from a cancer cell, wherein the method comprises administering the multispecific antigen-binding molecule of any one of [1] to [18].

Further, the present invention also provides the following as other examples:
[46] the composition of [42], the use of [43], the multispecific antigen-binding molecule of [44], or the method of [45], which is used in combination with an activator of antigen-presenting cells;
[47] a pharmaceutical composition for use in combination with an activator of antigen-presenting cells, which comprises the multispecific antigen-binding molecule of any one of [1] to [18];
[48] a pharmaceutical composition for use in combination with the multispecific antigen-binding molecule of any one of [1] to [18], which comprises an activator of antigen-presenting cells;
[49] a pharmaceutical composition comprising the multispecific antigen-binding molecule of any one of [1] to [18] and an activator of antigen-presenting cells;
[50] the pharmaceutical composition of any one of [47] to [49], which is a composition for inducing an antitumor immune response, a composition for activating cytotoxic T cells, a composition for inducing cytotoxicity, a composition for suppressing cell proliferation, a composition for activating immunity against cancer cells or cancer tissue containing cancer cells, a composition for preventing or treating cancer, a composition for treating an individual having cancer, or a composition for allowing an MHC class I protein of an antigen-presenting cell to present an antigen peptide derived from cancer cells;
[51] a kit comprising the multispecific antigen-binding molecule of any one of [1] to [18] and an activator of antigen-presenting cells;
[52] the pharmaceutical composition of any one of [47] to [50] or the kit of [51], wherein the activator of antigen-presenting cells is interferon α (IFNa), poly I:C (poly-I:C), or a T cell activator; and
[53] the pharmaceutical composition or kit of [52], wherein the T cell activator is a bispecific antibody that induces cytotoxic activity by crosslinking a T cell and a cancer cell, or an antibody or an agent that activates T cells.

### [Effects of the Invention]

In one embodiment, a multispecific antigen-binding molecule of the present invention can crosslink antigen-presenting cells such as dendritic cells to target cells to promote phagocytosis of target cells by the antigen-presenting cells.

Further, in one embodiment, a multispecific antigen-binding molecule of the present invention is capable of making antigen-presenting cells phagocytose target cells as they are without subjecting target cells to disruption, so that not only cell surface antigens of the target cells but also their intracellular antigens are presented by the antigen-presenting cells, enabling the presentation of a wide variety of antigens by antigen-presenting cells.

### [Brief Description of Drawings]

Fig. 1 is a schematic diagram showing an example of an antibody that crosslinks an antigen-presenting cell and a target cell. Fig 1(A) shows that a bispecific antibody crosslinks an antigen-presenting cell to a target cell and promotes phagocytosis of the target cell. Fig. 1(B) shows that an antigen-presenting cell that has phagocytosed a target cell presents various target cell-derived antigens on MHC and activates target cell-reactive effector cells.

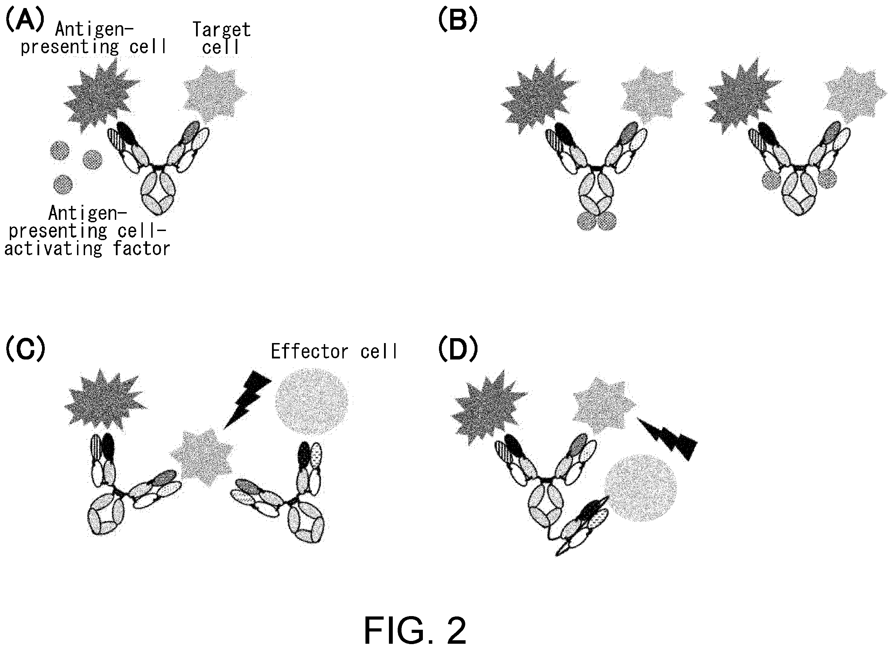

Fig. 2 is a schematic diagram showing examples of methods for enhancing the efficiency of antigen presentation by antigen-presenting cells. Fig. 2(A) shows a method in which a bispecific antibody that crosslinks an antigen-presenting cell and a target cell is used in combination with an antigen-presenting cell-activating factor. Fig. 2(B) shows methods of conjugating an antigen-presenting cell-activating factor to a bispecific antibody that crosslinks an antigen-presenting cell and a target cell. Fig. 2(C) shows a method in which a bispecific antibody that crosslinks an antigen-presenting cell to a target cell is used in combination with an antibody that induces a cytotoxic activity against the target cell to activate the antigen-presenting cell by inflammatory cytokines and such released from dead cells. Fig. 2(D) shows a method in which an effector cell-activating antibody fragment or agent is conjugated to a bispecific antibody that crosslinks an antigen-presenting cell and target cell to activate the antigen-presenting cell by inflammatory cytokines and such released from dead cells. The conjugate site shown in the figure is an example, and the site is not limited thereto.
Fig. 3 shows the result of evaluating CLEC9A expression level in dendritic cells by flow cytometry.
Fig. 4 is a graph showing the phagocytosis ratio of target cancer cells by CD103-positive or -negative dendritic cells.
Fig. 5 is a graph showing the phagocytosis ratio of target cancer cells by CD103-positive or -negative dendritic cells.
Fig. 6 shows the result of measuring the amount of mIFNy in a culture supernatant after co-culturing target cells, dendritic cells, and CD8⁺ T cells in the presence of the bispecific antibodies prepared in Example 2 and poly I:C (Fig. 6(A)) or IFNα (Fig. 6(B)).
Fig. 7 shows the change over time in tumor diameter in tumor-transplanted mice to which the bispecific antibodies prepared in Example 2 and poly I:C were administered.
Fig 8 shows the result of evaluating the CD40 expression level in CD103-positive dendritic cells by flow cytometry, after co-culturing target cells and dendritic cells in the presence of the bispecific antibodies prepared in Example 2 and poly I:C.
Fig 9 shows the result of evaluating CD40 expression level in CD103-positive dendritic cells by flow cytometry after co-culturing target cells, dendritic cells, and T cells in the presence of the bispecific antibodies prepared by the method described in Example 2 and a bispecific antibody of the anti-GPC3 antibody and the anti-CD3 antibody(GPC3//CD3).
Fig. 10 is a graph showing the phagocytosis ratio of target cancer cells expressing hEpCAM by CD103-positive or -negative dendritic cells.
Fig. 11 shows the result of measuring the amount of mIFNy in a culture supernatant after co-culturing target cells, dendritic cells, and CD8⁺ T cells in the presence of a bispecific antibody that binds to DEC205 and GPC3, and poly I:C.
Fig. 12 shows the result of measuring the amount of mIFNy in a culture supernatant after co-culturing target cells, dendritic cells, and CD8⁺ T cells in the presence of a bispecific antibody that binds to Clec9a and hEpCAM, and poly I:C.

### [Description of Embodiments]

The following definitions are provided to facilitate understanding of the present invention described herein.

As one aspect of the present invention, a multispecific antigen binding molecule comprising a first antigen-binding domain that binds to a first antigen on an antigen-presenting cell and a second antigen-binding domain that binds to a second antigen on a target cell is provided. In one embodiment, the multispecific antigen-binding molecule can crosslink antigen-presenting cells such as dendritic cells to target cells, and promote phagocytosis of target cells by the antigen-presenting cells. In one embodiment, the antigen-presenting cells are capable of presenting to T cells multiple types of antigen peptides derived from target cells that have been engulfed by phagocytosis. As a result, for example, activation of CD8⁺ effector T cells (cytotoxic T cells) targeting multiple types of tumor antigens derived from target cells can be induced. Therefore, in one embodiment, by using the multispecific antigen-binding molecule, it is possible to provide a cancer treatment having a higher efficacy as compared to conventional cancer immunotherapy targeting a specific (typically one type of) tumor antigen. Moreover, in a specific embodiment, an antitumor effect is induced in a subject administered with the multispecific antigen-binding molecule.

### Antigen-binding molecule

In the present invention, the term "antigen-binding molecule" is used in the broadest sense indicating a molecule comprising an antigen-binding domain; and specifically, it includes various types of molecules as long as they show antigen-binding activity.
Molecules in which an antigen-binding domain is linked to an FcRn-binding domain include, for example, antibodies. Antibodies may include single monoclonal antibodies (including agonistic antibodies and antagonistic antibodies), human antibodies, humanized antibodies, chimeric antibodies, and such. Alternatively, when used as antibody fragments, they preferably include antigen-binding domains and antigen-binding fragments (for example, Fab, F(ab')2, scFv, and Fv). Scaffold molecules where three dimensional structures, such as already-known stable α/β barrel protein structure, are used as a scaffold (base) and only some portions of the structures are made into libraries to construct antigen-binding domains are also included in antigen-binding molecules of the present invention.

Herein, an "antigen-binding domain" may be of any structure as long as it binds to an antigen of interest. Such domains preferably include, for example:
antibody heavy-chain and light-chain variable regions;
a module of about 35 amino acids called A domain which is contained in an *in vivo* cell membrane protein, Avimer (WO 2004/044011, WO 2005/040229);
Adnectin containing the 10Fn3 domain which binds to the protein moiety of fibronectin, a glycoprotein expressed on cell membrane (WO 2002/032925);
Affibody which uses as scaffold an IgG-binding domain of Protein A forming a 58-amino acid three-helix bundle (WO 1995/001937);
Designed Ankyrin Repeat proteins (DARPins) which are a region exposed on the molecular surface of ankyrin repeats (AR) having a structure in which a subunit consisting of a turn comprising 33 amino acid residues, two antiparallel helices, and a loop is repeatedly stacked (WO 2002/020565);
Anticalins and such, which are regions consisting of four loops that support one side of a barrel structure composed of eight antiparallel strands twisted toward the center that are highly conserved among lipocalin molecules such as neutrophil gelatinase-associated lipocalin (NGAL) (WO 2003/029462); and
the concave region formed by the parallel-sheet structure inside the horseshoe-shaped structure constituted by stacked repeats of the leucine-rich-repeat (LRR) module of the variable lymphocyte receptor (VLR) which does not have the immunoglobulin structure as the system of acquired immunity in jawless vertebrate such as lamprey and hagfish (WO 2008/016854). Suitable examples of the antigen-binding domains of the present invention include antigen-binding domains comprising antibody heavy-chain and light-chain variable regions.

In the present invention, the "antigen-binding molecule" is not particularly limited as long as the molecule comprises an "antigen-binding domain" of the present invention. The antigen-binding molecule may further comprise a peptide or a protein having a length of approximately 5 amino acids or more. The peptide or the protein is not limited to a peptide or a protein derived from an organism, and may be, for example, a polypeptide consisting of an artificially designed sequence. Also, a natural polypeptide, a synthetic polypeptide, a recombinant polypeptide, or the like may be used.

Preferred examples of the antigen-binding molecules of the present invention can include an antigen-binding molecule comprising an FcRn-binding domain comprised in an antibody Fc region. As method for extending the blood half-life of proteins administered *in vivo,* methods which add an antibody FcRn-binding domain to a protein of interest to use the FcRn-mediated recycling function are well known.

FcRn-binding domains of the present invention may be in particular either those with reduced binding activity toward an Fcγ receptor, or those having increased binding activity toward an Fcγ receptor. In this context, the Fcγ receptor (also referred to as FcyR or FcgR herein) refers to a receptor capable of binding to the Fc region of IgG1, IgG2, IgG3, or IgG4 and means any member of the family of proteins substantially encoded by Fcγ receptor genes. In humans, this family includes, but is not limited to: FcyRI (CD64) including isoforms FcyRIa, FcyRIb, and FcyRIc; FcyRII (CD32) including isoforms FcyRIIa (including allotypes H131 (H type) and R131 (R type)), FcyRIIb (including FcyRIIb-1 and FcyRIIb-2), and FcyRIIc; and FcyRIII (CD16) including isoforms FcyRIIIa (including allotypes V158 and F158) and FcyRIIIb (including allotypes FcγRIIIb-NA1 and FcyRIIIb-NA2); and any yet-to-be-discovered human FcyR or FcyR isoform or allotype. The FcyR includes those derived from humans, mice, rats, rabbits, and monkeys. The FcyR is not limited to these molecules and may be derived from any organism. The mouse FcyRs include, but are not limited to, FcyRI (CD64), FcyRII (CD32), FcyRIII (CD16), and FcyRIII-2 (CD16-2), and any yet-to-be-discovered mouse FcyR or FcyR isoform or allotype. Preferred examples of such Fcγ receptors include human FcyRI (CD64), FcyRIIa (CD32), FcyRIIb (CD32), FcyRIIIa (CD16), and/or FcyRIIIb (CD16).

Reduced binding activity against an Fcγ receptor can be confirmed by a well-known method such as FACS, ELISA format, ALPHAScreen (amplified luminescent proximity homogeneous assay screen), or the BIACORE method based on a surface plasmon resonance (SPR) phenomenon (Proc. Natl. Acad. Sci. USA (2006) 103 (11), 4005-4010).

A "first antigen-binding domain that binds to a first antigen on an antigen-presenting cell" and a "second antigen-binding domain that binds to a second antigen on a target cell" (hereinafter, these binding domains are collectively referred to as antigen binding domains) contained in a multispecific antigen-binding molecule of the present invention mean a region that specifically binds to the respective antigen, which is a first antigen on an antigen presenting cell, or a second antigen on a target cell. Examples of the binding domains include a region containing an antigen-binding region of an antibody. When selecting a first antigen on the antigen-presenting cell and a second antigen on the target cell to which a multispecific antigen-binding molecule of the present invention binds, the antigens are selected to be mutually different. When the molecular weight of an antigen is large, the antigen-binding region of the antibody can only bind to a specific part of the antigen. The specific part is called an epitope. The antigen binding domains may be provided by one or more antibody variable domains. In a preferred embodiment, the antigen binding domains comprise an antibody light chain variable region (VL) and an antibody heavy chain variable region (VH).

An "antigen-binding molecule" of the present invention may be an antibody fragment that contains both heavy and light chains forming the "antibody variable regions" of the present invention within a single polypeptide chain, but lacking the constant regions. Such antibody fragments may be, for example, Fv, Fab, Fab', Fab'-SH, F(ab')2; diabodies; linear antibodies; single-chain antibody molecules (for example, scFv (single chain Fv), sc(Fv)₂, sc(Fab')₂); and single domain antibodies (e.g., VHH, VH, VL).

A "multispecific" antigen-binding molecule refers to an antigen-binding molecule having in the same molecule two or more variable regions that specifically recognize different epitopes. A preferred example of a multispecific antigen-binding molecule of the present invention is an antigen-binding molecule having in the same molecule two variable regions that specifically recognize different epitopes.

In the present invention, "specifically binds" means binding in a state where one of the molecules involved in specific binding does not show any significant binding to molecules other than a single or a number of binding partner molecules. Furthermore, it is also used when an antigen-binding domain is specific to a particular epitope among multiple epitopes contained in an antigen. When an epitope bound by an antigen-binding domain is contained in multiple different antigens, antigen-binding molecules comprising the antigen-binding domain can bind to various antigens that have the epitope.

Depending on a "first antigen on an antigen-presenting cell" to be targeted, one skilled in the art can appropriately select a heavy chain variable region and a light chain variable region that bind to the antigen as a heavy chain variable region and a light chain variable region contained in a "first antigen-binding domain that binds to a first antigen on an antigen-presenting cell" in a multispecific antigen-binding molecule of the present invention. Similarly, depending on a "second antigen on a target cell" to be targeted, one skilled in the art can appropriately select a heavy chain variable region and a light chain variable region that bind to the antigen as a heavy chain variable region and a light chain variable region contained in a "second antigen-binding domain that binds to a second antigen on a target cell" in a multispecific antigen-binding molecule of the present invention. As examples of a "first antigen-binding domain that binds to a first antigen on an antigen-presenting cell" in a multispecific antigen-binding molecule of the present invention, the following can be given, but are not restricted thereto:
a CLEC9A-binding domain having:
   (1) a heavy chain variable region comprising the CDR1 sequence set forth in SEQ ID NO: 14, the CDR2 sequence set forth in SEQ ID NO: 15, and the CDR3 sequence set forth in SEQ ID NO: 16, and a light chain variable region comprising the CDR1 sequence set forth in SEQ ID NO: 18, the CDR2 sequence set forth in SEQ ID NO: 19, and the CDR3 sequence set forth in SEQ ID NO: 20;
   (2) a heavy chain variable region having the amino acid sequence set forth in SEQ ID NO: 17 and a light chain variable region having the amino acid sequence set forth in SEQ ID NO: 21; or
   (3) a heavy chain having the amino acid sequence set forth in SEQ ID NO: 1 and a light chain having the amino acid sequence set forth in SEQ ID NO: 2;
   as well as a DEC205-binding domain having:
   (1') a heavy chain variable region comprising the CDR1 sequence set forth in SEQ ID NO: 30, the CDR2 sequence set forth in SEQ ID NO: 31, and the CDR3 sequence set forth in SEQ ID NO: 32; and a light chain variable region comprising the CDR1 sequence set forth in SEQ ID NO: 34, the CDR2 sequence set forth in SEQ ID NO: 35, and the CDR3 sequence set forth in SEQ ID NO: 36;
   (2') a heavy chain variable region having the amino acid sequence set forth in SEQ ID NO: 33 and a light chain variable region having the amino acid sequence set forth in SEQ ID NO: 37; or
   (3') a heavy chain having the amino acid sequence set forth in SEQ ID NO: 7 and a light chain having the amino acid sequence set forth in SEQ ID NO: 8.

As examples of a "second antigen-binding domain that binds to a second antigen on a target cell" in a multispecific antigen-binding molecule of the present invention, the following can be given, but are not restricted thereto:
a GPC3-binding domain having:
   (4) a heavy chain variable region comprising the CDR1 sequence set forth in SEQ ID NO: 22, the CDR2 sequence set forth in SEQ ID NO: 23, and the CDR3 sequence set forth in SEQ ID NO:24, and a light chain variable region comprising the CDR1 sequence set forth in SEQ ID NO: 26, the CDR2 sequence set forth in SEQ ID NO: 27, and the CDR3 sequence set forth in SEQ ID NO: 28;
   (5) a heavy chain variable region having the amino acid sequence set forth in SEQ ID NO: 25 and a light chain variable region having the amino acid sequence set forth in SEQ ID NO: 29; or
   (6) a heavy chain having the amino acid sequence set forth in SEQ ID NO: 3 and a light chain having the amino acid sequence set forth in SEQ ID NO: 4;
as well as an EpCAM-binding domain having:
   (4') a heavy chain variable region comprising the CDR1 sequence set forth in SEQ ID NO: 22, the CDR2 sequence set forth in SEQ ID NO: 23, and the CDR3 sequence set forth in SEQ ID NO: 24, and a light chain variable region comprising the CDR1 sequence set forth in SEQ ID NO: 26, the CDR2 sequence set forth in SEQ ID NO: 27, and the CDR3 sequence set forth in SEQ ID NO: 28;
   (5') a heavy chain variable region having the amino acid sequence set forth in SEQ ID NO: 25 and a light chain variable region having the amino acid sequence set forth in SEQ ID NO: 29; or
   (6') a heavy chain having the amino acid sequence set forth in SEQ ID NO: 3 and a light chain having the amino acid sequence set forth in SEQ ID NO: 4.

Antibody variable regions used to produce various binding domains of antigen-binding molecules described herein are generally formed by three complementarity-determining regions (CDRs) that are separated by four framework regions (FRs). CDRs are regions that substantially determine the binding specificity of an antibody. The amino acid sequences of CDRs are highly diverse. On the other hand, the amino acid sequences constituting FRs often have high identity even among antibodies with different binding specificities. Therefore, generally, the binding specificity of a given antibody can be transplanted to another antibody by CDR grafting.

In the present specification, the "first antigen on the antigen-presenting cell" may preferably be an antigen that is specifically expressed on the cell surface of an antigen-presenting cell, for example, an antigen that is specifically expressed on dendritic cells. In one embodiment, it is an antigen that is specifically expressed on the dendritic cell subset responsible for cross-presentation. Examples of antigens on dendritic cells include CD1a, CD1c, CD11b, CD11c, FcεR1, CD141, XCR1, IgE, CD14, CD163, CD123, BDCA-2, ChemR23, Clec9a, CD206, CD207, and CD209 (Front Immunol. 2014 Apr 1; 5:131), and of these antigens on dendritic cells, those classified as C-type lectin receptors include DEC205, Dectin-1, Dectin-2, Mincle, CD206, CD207 (Langerin), CD209 (DC-SIGN), Clec9a (DNGR1), Clec10a (CD301, MGL), and Clec12a (J Leukoc Biol. 2017 Oct; 102(4): 1017-1034). However, antigens on dendritic cells are not necessarily limited to these.

In the present specification, the "second antigen on the target cell" may preferably be an antigen which is specifically expressed on the surface of a target cell. In the present specification, the "target cell" is not particularly limited as long as it is a cell that is phagocytosed by an antigen-presenting cell and cross-presented, and examples thereof include cancer cells, bacteria, and cells infected with viruses and the like. "Cancer-specific antigen" as a non-limiting example of an antigen on a target cell means an antigen expressed by a cancer cell, which enables distinguishing between a cancer cell and a healthy cell; for example, it includes antigens that are expressed as cells become malignant and abnormal sugar chains that appear on the cell surface or on protein molecules when the cells become cancerous. Specific examples thereof include ALK receptor (pleiotrophin receptor), pleiotrophin, KS 1/4 pancreatic cancer antigen, ovary cancer antigen (CA125), prostatic acid phosphate, prostate-specific antigen (PSA), melanoma-associated antigen p97, melanoma antigen gp75, high-molecular-weight melanoma antigen (HMW-MAA), prostate-specific membrane antigen, carcinoembryonic antigen (CEA), polymorphic epithelial mucin antigen, human milk fat globule antigen, colorectal tumor-associated antigen (e.g., CEA, TAG-72, CO17-1A, GICA 19-9, CTA-1, and LEA), Burkitt's lymphoma antigen 38.13, CD19, human B lymphoma antigen CD20, CD33, melanoma-specific antigen (e.g., ganglioside GD2, ganglioside GD3, ganglioside GM2, and ganglioside GM3), tumor-specific transplantation antigen (TSTA), T antigen, virus-induced tumor antigen (e.g., envelope antigens of DNA tumor virus and RNA tumor virus), colon CEA, oncofetal antigen α-fetoprotein (e.g., oncofetal trophoblastic glycoprotein 5T4 and oncofetal bladder tumor antigen), differentiation antigen (e.g., human lung cancer antigens L6 and L20), fibrosarcoma antigen, human T cell leukemia-associated antigen Gp37, newborn glycoprotein, sphingolipid, breast cancer antigen (e.g., EGFR (epithelial growth factor receptor)), NY-BR-16, NY-BR-16 and HER2 antigen (p185HER2), polymorphic epithelial mucin (PEM), malignant human lymphocyte antigen APO-1, differentiation antigen such as I antigen found in fetal erythrocytes, primary endoderm I antigen found in adult erythrocytes, I (Ma) found in embryos before transplantation or gastric cancer, M18, M39 found in mammary gland epithelium, SSEA-1 found in bone marrow cells, VEP8, VEP9, Myl, VIM-D5, D156-22 found in colorectal cancer, TRA-1-85 (blood group H), SCP-1 found in testis and ovary cancers, C14 found in colon cancer, F3 found in lung cancer, AH6 found in gastric cancer, Y hapten, Ley found in embryonic cancer cells, TL5 (blood group A), EGF receptor found in A431 cells, E1 series (blood group B) found in pancreatic cancer, FC10.2 found in embryonic cancer cells, gastric cancer antigen, CO-514 (blood group Lea) found in adenocarcinoma, NS-10 found in adenocarcinoma, CO-43 (blood group Leb), G49 found in A431 cell EGF receptor, MH2 (blood group ALeb/Ley) found in colon cancer, 19.9 found in colon cancer, gastric cancer mucin, T5A7 found in bone marrow cells, R24 found in melanoma, 4.2, GD3, D1.1, OFA-1, GM2, OFA-2, GD2, and M1:22:25:8 found in embryonic cancer cells, SSEA-3 and SSEA-4 found in 4-cell to 8-cell embryos, cutaneous T cell lymphoma-associated antigen, MART-1 antigen, sialyl Tn (STn) antigen, colon cancer antigen NY-CO-45, lung cancer antigen NY-LU-12 variant A, adenocarcinoma antigen ART1, paraneoplastic associated brain-testis-cancer antigen (onconeuronal antigen MA2 and paraneoplastic neuronal antigen), neuro-oncological ventral antigen 2 (NOVA2), blood cell cancer antigen gene 520, tumor-associated antigen CO-029, tumor-associated antigen MAGE-C1 (cancer/testis antigen CT7), MAGE-B1 (MAGE-XP antigen), MAGE-B2 (DAM6), MAGE-2, MAGE-4a, MAGE-4b, MAGE-X2, cancer-testis antigen (NY-EOS-1), YKL-40, and any fragment of these polypeptides, and modified structures thereof (aforementioned modified phosphate groups, sugar chains, etc.), EpCAM, EREG, CA19-9, CA15-3, sialyl SSEA-1 (SLX), HER2, PSMA, CEA, and CLEC12A. As a cancer-specific antigen which is the binding target of a second antigen-binding domain contained in a multispecific antigen-binding molecule of the present invention, one that is expressed on the cell surface is particularly preferable, and such cancer-specific antigens include, for example, GPC3, IL6R, CD19, CD20, EGFR, HER2, EpCAM and EREG.

As one of the preferable embodiments of an "antigen-binding molecule" of the present invention, an antibody containing a variable region of an antibody of the present invention can be given.

### Antibodies

Herein, "antibody" refers to a natural immunoglobulin or an immunoglobulin produced by partial or complete synthesis. Antibodies can be isolated from natural sources such as naturally-occurring plasma and serum, or culture supernatants of antibody-producing hybridomas. Alternatively, antibodies can be partially or completely synthesized using techniques such as genetic recombination. Preferred antibodies include, for example, antibodies of an immunoglobulin isotype or subclass belonging thereto. Known human immunoglobulins include antibodies of the following nine classes (isotypes): IgG1, IgG2, IgG3, IgG4, IgA1, IgA2, IgD, IgE, and IgM. Of these isotypes, antibodies of the present invention include IgG1, IgG2, IgG3, and IgG4.

Methods for producing an antibody with desired binding activity are known to those skilled in the art, and antibodies can be obtained as polyclonal or monoclonal antibodies. Antibodies of the present invention preferably produced are monoclonal antibodies derived from mammals. Such mammal-derived monoclonal antibodies include antibodies produced by hybridomas or host cells transformed with an expression vector carrying an antibody gene by genetic engineering techniques.

As antibodies, recombinant antibodies produced by gene recombination technology can be used. Recombinant antibodies are obtained by cloning DNA encoding the same from hybridoma or antibody-producing cells such as sensitized lymphocytes that produce antibodies, incorporating it into a vector, introducing this into a host (host cell), and causing production therein.

Bispecific antibodies are not limited to the IgG type, but for example, an IgG type bispecific antibody can be secreted by a hybrid hybridoma (quadroma) produced by fusing two kinds of hybridomas that produce IgG antibodies (Milstein C et al. Nature 1983, 305: 537-540). In addition, it can be secreted by introducing into cells genes of L chains and H chains constituting two types of IgGs of interest, a total of four types of genes, and coexpressing them.

Antibodies of the present invention can be generated by methods known to those skilled in the art. Specifically, a DNA encoding an antibody of interest is integrated into an expression vector. In this case, it is integrated into an expression vector so that it is expressed under the control of expression control regions, for example, an enhancer and a promoter. Next, host cells are transformed with this expression vector and allowed to express antibodies. When doing so, an appropriate combination of hosts and expression vectors can be used.

Antibodies of the present invention thus obtained can be isolated from the inside or outside (medium etc.) of the host cells and purified as substantially pure and homogeneous antibodies. Antibodies can be isolated and purified using isolation and purification methods conventionally used for antibody purification, without limitation. For example, antibodies can be isolated and purified by appropriately selecting and combining column chromatography, filtration, ultrafiltration, salting-out, solvent precipitation, solvent extraction, distillation, immunoprecipitation, SDS-polyacrylamide gel electrophoresis, isoelectric focusing, dialysis, recrystallization, and such.

As one of the preferred embodiments of the multispecific antigen-binding molecules of the present invention, a multispecific antibody can be given. As the multispecific antibody of the present invention, a bispecific antibody is particularly preferable.

For association by which multispecific antibodies are formed, a technique that can be used is to suppress the unintended association between H chains by introducing electric charge repulsion to the interface between the second constant regions (CH2) or the third constant regions (CH3) of the antibody H chains (WO2006/106905).

An alternative technique known in the art can also be used for the association to form the multispecific antibodies of the present invention. An amino acid side chain present in the variable domain of one of the antibody H chains is substituted by a larger side chain (knob), and its partner amino acid side chain present in the variable domain of the other H chain is substituted by a smaller side chain (hole) such that the knob can be placed into the hole, which enables efficient association of polypeptides having Fc regions with different amino acids (WO1996/027011; Ridgway JB et al., Protein Engineering (1996) 9, 617-621; and Merchant AM et al. Nature Biotechnology (1998) 16, 677-681, US20130336973).

In addition to this technique, a further alternative technique known in the art may be used for forming the multispecific antibodies of the present invention. A portion of CH3 of one of the antibody H chains is converted to the corresponding IgA-derived sequence, and its complementary portion in CH3 of the other H chain is converted to the corresponding IgA-derived sequence. The resulting strand-exchange engineered domain CH3 can be used to cause efficient association between polypeptides having different sequences through complementary CH3 association (Protein Engineering Design & Selection, 23; 195-202, 2010). By use of this technique known in the art, the multispecific antibodies of interest can also be efficiently formed.

Even if the multispecific antibodies of interest cannot be formed efficiently, the multispecific antibodies of the present invention may be obtained by the separation and purification of the multispecific antibodies of interest from among produced antibodies. For example, a previously reported method involves introducing amino acid substitutions to two types of H chain variable regions to make their isoelectric points different, so that two types of homodimers and the heterodimerized antibodies of interest can be separately purified by ionexchange chromatography (WO2007114325). A method using protein A to purify a heterodimerized antibody consisting of a mouse IgG2a H chain which binds to protein A and a rat IgG2b H chain which does not bind to protein A has been reported to date as a method for purifying the heterodimers (WO98050431 and WO95033844). Alternatively, the interaction between each H chain and protein A is modified by using H chains in which amino acid residues at EU numbering positions 435 and 436 which constitute the binding site between protein A and IgG are substituted with amino acids having different binding strength toward protein A such as Tyr and His, and only the heterodimerized antibodies can be efficiently purified by using a protein A column.

Alternatively, a common L chain that can confer binding ability to several different H chains may be obtained and used as the common L chain of the multispecific antibodies. Efficient multispecific IgG expression is possible by introducing genes of such a common L chain and multiple different H chains into cells to express IgGs (Nature Biotechnology (1998) 16, 677-681). When selecting a common H chain, it is also possible to use a method of selecting a common L chain corresponding to any different H chain and exhibiting a high binding ability thereto (WO2004/065611).

It is also possible to use a plurality of these technologies, for example, a combination of two or more. In addition, these techniques can be appropriately applied separately to the two H chains to be associated. The antigen-binding molecules of the present invention may be separately prepared to have the same amino acid sequence based on those with the aforementioned modification.

A multispecific antigen-binding molecule of the present invention comprises a first antigen-binding domain that binds to a first antigen on an antigen-presenting cell and a second antigen-binding domain that binds to a second antigen on a target cell. Because of this, the antigen-presenting cell and target cell are crosslinked via the multispecific antigen-binding molecule of the present invention. Specifically, the multispecific antigen-binding molecule of the present invention simultaneously binds to an antigen on an antigen-presenting cell and a cancer antigen on a target cell to bring the antigen-presenting cell into close proximity to the target cell (it can also be described as recruiting, attracting, or targeting the antigen presenting cell to the target cell). As a result, phagocytosis of the target cell by the antigen presenting cell can be induced. Methods for detecting/measuring phagocytosis include those that make dendritic cells phagocytose labeled cells and detect/measure the label by flow cytometry, image/measure the label with a microscope, or detect/measure the label by a reporter assay.

"Undisrupted" cells mean that the cells are not in debris. An example of cells becoming cell debris is cell death of cancer cells. Cell death is roughly classified into necrosis and apoptosis based on the morphological and biochemical characteristics. Apoptotic cells shrink, form cell fragments called apoptotic bodies, and are phagocytosed and removed by phagocytes *in vivo.* On the other hand, necrosis causes inflammation of peripheral cells by swelling of cells and eventual leakage of cell contents out of the cells. Apoptosis and necrosis are both considered to be mechanisms by which cancer cells disappear due to anticancer agents and radiation therapy (YAKUGAKU ZASSHI 137(11) 1315-1321 (2017)).

As a general method for disrupting cells, ultrasonic treatment, use of a surfactant, osmotic shock method, disrupting with a homogenizer, disrupting with glass beads, and such are known (these methods have been exemplified in the website: https://www.gelifesciences.co.jp/technologies/protein_reparation/lysis.html).

In a preferred embodiment, it is desirable that, as the target cells to be phagocytosed, cells that are not disrupted and are countable are allowed to be phagocytosed by the antigen-presenting cells. By making antigen-presenting cells engulf undisrupted target cells as they are, it is possible to allow the antigen presenting cells to present a wide variety of antigens which the target cells have, including not only cell surface antigens, but also intracellular antigens.

Multiple types of antigens derived from target cells taken up into antigen presenting cells are processed, and the produced antigenic peptides are presented by the antigen presenting cells (epitope spreading). These antigen peptides are presented as a complex with MHC class II molecules to activate CD4⁺ T cells, and also presented as a complex with MHC class I molecules (cross-presentation) to activate CD8⁺ T cells. Methods for detecting/identifying/measuring antigen peptides derived from target cells presented on the MHC molecules of antigen presenting cells include: methods of evaluating whether or not an antigen presenting cell can activate T cells having a TCR responsive towards an arbitrary peptide antigen-MHC complex (for example, a system using OT-I mice (having OVA-reactive T cells) described in the Examples section of the present specification); methods of detecting/measuring by FACS an arbitrary antigen peptide-MHC complex using an antibody that specifically recognizes the complex; methods of directly detecting/measuring antigen peptides presented on MHCs by MS, and the like. Methods for detecting/measuring T cell activation include: methods of detecting or measuring by ELISA or FACS cytokines released upon activation; methods of detecting/measuring activation markers by FACS; methods of detecting/measuring increase in cell number, and the like.

T cells thus activated are T cells specific for the antigen peptide presented by the antigen presenting cell. By using a multispecific antigen-binding molecule of the present invention, multiple types of antigen peptides derived from target cells are presented on antigen-presenting cells, thus making it possible to induce the activation of T cells targeting multiple types of cancer antigen peptides. The multiple types of cancer antigen peptides also include those derived from antigens different from the second antigen on the target cell.

A suitable example of antigen-presenting cells having such a function is dendritic cells.

Dendritic cells are known to play an important role in antitumor immunity in both innate and adaptive immunity (Nature Reviews Cancer 4, 11-22 (January 2004)). In innate immunity, dendritic cells play a role in phagocytosing, engulfing, and degrading apoptotic cancer cells (phagocytosis). In adaptive immunity, dendritic cells present antigen peptides derived from cancer cells that have been taken up into the cells on major histocompatibility complexes (MHCs), activate naive T cells having T cell receptors (TCRs) that specifically bind to each, and the activated naive T cells are differentiated into cytotoxic T cells (CTLs), helper T cells and the like to induce an antitumor immune response (Nature Reviews Immunology volume 1, pages 126-134 (2001)). The antitumor immune response is an immune response consisting of innate immunity and adaptive immunity against cancer cells, and it is considered that cytotoxic T cells particularly play a central role in the antitumor immune response.

Cross-presentation refers to the presentation of a peptide (antigen peptide) resulted from incorporation and processing of an antigen derived from another cell to CD8⁺ cytotoxic T cells as a complex with an MHC class I protein. Methods for measuring cross-presentation include methods of recognizing the antigen peptide MHC-I on antigen-presenting cells by flow cytometry or mass cytometry, methods of measuring activation of CD8⁺ T cells by co-culturing an arbitrary antigen peptide MHC-I-reactive CD8⁺ cytotoxic T cells and antigen presenting cells presenting this antigen, and the like. Methods for measuring CD8⁺ T cell activation include: methods of measuring IFNγ, granzyme B, perforin, and such released upon activation by ELISA, ELISpot assay, flow cytometry, and such; methods of measuring an activation marker on T cells (CD25, CD69, etc., Clin Cham Acta. 2012 Sep 8; 413(17-18):1338-49) by flow cytometry; methods of measuring a change in T cell proliferation ability; and the like. It is known that a specific subset of dendritic cells plays the role of cross-presentation and activation (cross-priming) of T cells. CLEC9A (C-type lectin 9A), DEC205 (dendritic and epithelial cells, 205 kDa), CD207 (Cluster of Differentiation 207), XCR1 (XC-chemokine receptor 1), TLR3 (Toll-like receptor 3), and the like are known as markers of dendritic cells responsible for cross-presentation (Nat Rev Immunol. 2011, 11(9), 575-83).

It is known that CLEC9A, which is expressed on the cell surface of the dendritic cell subset responsible for cross-presentation, binds to F-actin exposed on necrotic cells and activates the intracellular signal transduction pathway through the interaction between intracellular domain containing ITAM (Immunoreceptor tyrosine-based activation motif) and Syk, and promotes cross-presentation by dendritic cells (Nat Rev Immunol. 2010 Jun; 10(6):387-402). Expression of DEC205 and the like is also known in the dendritic cell subset that is responsible for cross-presentation, but CLEC9A has been reported to be expressed in that subset with a specificity higher than DEC205 (Blood, 2012, vol. 119: pp.2284-2292).

In addition, by using a multispecific antigen-binding molecule of the present invention in combination with an activator of antigen-presenting cells, or in a form conjugated with an activator of antigen-presenting cells, it is possible to enhance the phagocytosis of target cells by antigen-presenting cells induced by crosslinking of antigen-presenting cells and target cells via the multispecific antigen-binding molecule of the present invention, and thus enhance the induction of cytotoxic activity against the target cells.

Conjugates of a multispecific antigen-binding molecule of the present invention and an antigen-presenting cell activator may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). A linker may be a "cleavable linker" facilitating release of the antigen-presenting cell activator in the cell. For example, an acid-labile linker, peptidase-sensitive linker, photolabile linker, dimethyl linker, or disulfide-containing linker (Chari et al., Cancer Res. 52:127-131 (1992)) may be used.

The conjugates of the present invention contemplate, but are not limited to such conjugates prepared with crosslinking reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate), which are commercially available (e.g., Thermo Fisher Scientific Inc.).

As antigen-presenting cell activators, agents that directly activate antigen-presenting cells (Figs. 2A and B) can be used, in addition to agents that activate immune cells other than antigen-presenting cells (Figs. 2C and D). When the latter is used, antigen-presenting cells can be indirectly activated by immune cells such as effector cells activated by the latter or by inflammatory cytokines and the like released from damaged dead cells. Examples of such activators include interferon α (IFNa), poly I:C (poly-I:C), T cell activators, and the like.

In one embodiment, the addition of these activators allows undisrupted target cells to be efficiently phagocytosed by antigen-presenting cells as they are, as compared to the case where they are not added. When undisrupted target cells are presented to antigen-presenting cells, it is possible to allow the antigen-presenting cells to present the antigens possessed by the target cells, including a wide variety ranging from not only cell surface antigens, but also intracellular antigens. As a result, antigen-presenting cells can be more effectively activated.

In addition, known adjuvants that activate dendritic cells include killed mycobacteria and LPS (lipopolysaccharide), aluminum hydroxide, aluminum phosphate (Molecular and Cellular Immunology, 7th edition, Elsevier Japan, 2014), and IFNα, IFNγ, TNFα, IL-1β, IL-6, and LL37-DNA complex (N Engl J Med 2009; 361:496-509). Also, agonists for TLR (Toll-like receptor), NLR (nucleotide-binding oligomerization domain-like (NOD-like) receptor), RLR (RIG-I-like receptor), and C-type lectin receptors are also known to act as activators of dendritic cells (Front Immunol. 2014; 5: 255).

By conjugating an agent that directly activates antigen-presenting cells to a multispecific antigen-binding molecule of the present invention, the antigen-presenting cells bound by the multispecific antigen-binding molecule of the present invention can be specifically activated (Fig. 2B). The agent may be conjugated to, for example, the Fc region or Fab region of the multispecific antigen-binding molecule of the present invention.

When using as a T cell activator an antigen-binding molecule having a binding site for an antigen that is specifically expressed in cells expressing a cancer-specific antigen to which a multispecific antigen-binding molecule of the present invention binds, and a binding site for a T cell surface antigen (e.g. CD3), in combination with the multispecific antigen-binding molecule of the present invention, the cancer cells to which the multispecific antigen-binding molecule of the present invention binds release inflammatory cytokines and the like upon being damaged by T cells recruited in the vicinity of the cancer cells mediated by the T cell activator, which enables specific activation of the antigen-presenting cells to which the multispecific antigen-binding molecule of the present invention binds (Fig. 2C). Examples of such T cell activators include bispecific antibodies that crosslink T cells and cancer cells to thereby induce cytotoxic activity against the cancer cells as described in WO2012073985.

In addition, by conjugating an antibody fragment or agent that activates effector cells (cytotoxic T cells) to a multispecific antigen-binding molecule of the present invention as a T cell activator, cancer cells to which the multispecific antigen-binding molecule of the present invention binds release inflammatory cytokines and the like upon being damaged by effector cells recruited in the vicinity of the cancer cells mediated by the antigen binding molecule, which enables specific activation of the antigen-presenting cells to which the multispecific antigen-binding molecule of the present invention binds (Fig. 2D). The T cell activator may be conjugated to, for example, the Fc region or Fab region of the multispecific antigen-binding molecule of the present invention.

The present invention also relates to a method for inducing an antitumor immune response, a method for activating cytotoxic T cells, a method for inducing cytotoxicity, a method for suppressing cell proliferation, a method for activating immunity against target cells or cancer tissues containing target cells, a method for preventing or treating cancer, a method for treating an individual having cancer, and a method of allowing an antigen peptide derived from a target cell to be presented on an MHC class I protein of an antigen presenting cell, which comprise a step of administering a multispecific antigen-binding molecule of the present invention to a subject (patient, subject, individual, etc.). Subjects include humans or non-human animals such as mice, rats, monkeys, rabbits, and dogs.

The present invention also relates to polynucleotides encoding an antigen-binding molecule of the present invention. A polynucleotide of the present invention can be incorporated into an arbitrary expression vector. A suitable host can be transformed with the expression vector to obtain cells expressing the antigen-binding molecule. An antigen binding molecule encoded by the polynucleotide can be obtained by culturing cells expressing the antigen-binding molecule and recovering the expression product from the culture supernatant. In other words, the present invention relates to a vector containing a polynucleotide encoding an antigen-binding molecule of the present invention, a cell carrying the vector, and a method of manufacturing the antigen-binding molecule comprising culturing the cell and recovering the antigen-binding molecule from the culture supernatant.

### Pharmaceutical compositions

In another aspect, the present invention provides pharmaceutical compositions (pharmaceutical preparations) comprising a multispecific antigen-binding molecule of the present invention as an active ingredient. The present invention also relates to compositions for inducing an antitumor immune response, pharmaceutical compositions for inducing the activation of cytotoxic T cells (cytotoxic T cell activators), pharmaceutical compositions for inducing cytotoxicity (therapeutic agents for inducing cytotoxicity), cell proliferation suppressors, and anticancer agents, which comprise the antigen-binding molecule as an active ingredient. The pharmaceutical compositions of the present invention can also be used as cancer therapeutic agents or cancer preventive agents (compositions for treating cancer or compositions for preventing cancer). The compositions for inducing an anti-tumor immune response, the cytotoxic T cell activators, the cytotoxicity inducing therapeutic agents, the cell proliferation suppressors, and the anti-cancer agents of the present invention are preferably administered to a subject suffering from cancer or a subject with a potential of relapse. In the context of the compositions of the present invention, the induction of an antitumor immune response may preferably be the activation of cytotoxic T cells (cytotoxic T lymphocytes; CTLs).

Further, in the present invention, the compositions for inducing an anti-tumor immune response, the cytotoxic T cell activators, the cytotoxicity-inducing therapeutic agents, the cell proliferation suppressors or anticancer agents, which comprise a multispecific antigen-binding molecule of the present invention as an active ingredient can be expressed as a method of inducing an antitumor immune response, a method of activating cytotoxic T cells, a method of inducing cytotoxicity, a method of suppressing cell proliferation, a method of activating immunity against cancer cells or tumor tissues containing cancer cells, or a method of preventing or treating cancer, which includes a step of administering the antigen-binding molecule to a subject. Alternatively, they can be expressed as a use of the antigen-binding molecule in the manufacture of a composition for inducing an antitumor immune response, a pharmaceutical composition for inducing the activation of cytotoxic T cells, a pharmaceutical composition for inducing cytotoxicity, a cell proliferation suppressor or an anticancer agent. Alternatively, they can also be expressed as the antigen-binding molecule for use in the induction of an anti-tumor immune response, induction of the activation of cytotoxic T cells, induction of cytotoxicity, suppression of cell proliferation, activation of immunity against cancer cells or tumor tissues containing cancer cells, or treatment or prevention of cancer. The compositions or pharmaceutical preparations may include a pharmaceutically acceptable carrier, and optionally, additional therapeutic agents. Moreover, the methods may optionally include a step of administering an additional therapeutic agent.

In the present invention, "comprising an antigen-binding molecule as an active ingredient" means containing the antigen-binding molecule as the main active ingredient, and does not limit the content ratio of the antigen-binding molecule.

Furthermore, the pharmaceutical compositions of the present invention, or compositions for inducing an antitumor immune response, pharmaceutical compositions for inducing cytotoxicity, cell proliferation suppressors and anticancer agents (hereinafter, pharmaceutical compositions or the like) can be used in combination with an activator of antigen presenting cells. Use of an activator of antigen presenting cells in combination with a pharmaceutical composition or the like containing a multispecific antigen-binding molecule of the present invention enables enhancement of the phagocytosis of target cells by antigen presenting cells, and thus enables enhancement of the cytotoxic effect against target cells. Examples of activators of antigen presenting cells include interferon α (IFNa), poly I:C (poly-I:C), T cell activators, and the like. As used herein, the phrase "using an activator of antigen-presenting cells in combination" means that the activator of antigen-presenting cells may be formulated together in a pharmaceutical composition or the like containing a multispecific antigen-binding molecule of the present invention, or the activator of antigen-presenting cells may be contained in a pharmaceutical composition or the like that is different from the pharmaceutical composition or the like containing the multispecific antigen-binding molecule of the present invention. Their dosage forms may be the same or different. When a multispecific antigen-binding molecule of the present invention and an activator of antigen-presenting cells are contained in different pharmaceutical compositions or the like, these pharmaceutical compositions or the like can be administered to a subject at the same time, or separately. Furthermore, these pharmaceutical compositions and the like may be provided as a kit.

Further, a multispecific antigen-binding molecule of the present invention or a pharmaceutical composition comprising a multispecific antigen-binding molecule of the present invention as an active ingredient can be used as a pharmaceutical composition for enhancing the phagocytosis of target cells by antigen-presenting cells, enhancing induction of their cytotoxic activity, or enhancing cytotoxic activity when used in combination with an antigen presenting cell activator or a pharmaceutical composition or such comprising an antigen presenting cell activator as an active ingredient. Further, an activator of an antigen-presenting cell or a pharmaceutical composition or such comprising an activator of an antigen-presenting cell as an active ingredient can be used as a pharmaceutical composition for enhancing the phagocytosis of target cells by antigen-presenting cells, enhancing induction of their cytotoxic activity, or enhancing cytotoxic activity when used in combination with a multispecific antigen-binding molecule of the present invention or a pharmaceutical composition comprising a multispecific antigen-binding molecule of the present invention as an active ingredient.

Herein, "used in combination" includes the case where a pharmaceutical composition or such comprising a multispecific antigen-binding molecule of the present invention as an active ingredient and a pharmaceutical composition or such comprising an activator of antigen-presenting cells as an active ingredient are simultaneously administered to a subject, and the case where they are separately administered to a subject. Their dosage forms may be the same or different. Furthermore, these pharmaceutical compositions or such may be provided as a kit.

Furthermore, the present invention provides a method that utilizes the effects produced by using a multispecific antigen-binding molecule of the present invention described above or a pharmaceutical composition or such comprising the antigen-binding molecule as an active ingredient in combination with an antigen presenting cell activator or a pharmaceutical composition or such comprising the antigen presenting cell activator as an active ingredient to enhance the cytotoxic activity or antitumor effect of the multispecific antigen-binding molecule of the present invention or the pharmaceutical composition or such comprising the multispecific antigen-binding molecule of the present invention as an active ingredient by the antigen presenting cell activator or the pharmaceutical composition or such comprising the antigen presenting cell activator as an active ingredient, .

The pharmaceutical compositions, compositions for inducing an antitumor immune response, cell proliferation suppressors, or anticancer agents of the present invention may be administered either orally or parenterally to patients. Preferred administration may be parental administration. Specifically, such administration methods include injection, nasal administration, transpulmonary administration, and percutaneous administration. Injections include, for example, intravenous injections, intramuscular injections, intraperitoneal injections, and subcutaneous injections. For example, pharmaceutical compositions, compositions for inducing an antitumor immune response, therapeutic agents for inducing cellular cytotoxicity, cell proliferation suppressors, or anticancer agents of the present invention can be administered locally or systemically by injection. Furthermore, appropriate administration methods can be selected according to the patient's age and symptoms. The dose can be selected, for example, from the range of 0.0001 mg to 1,000 mg per kg of body weight for each administration. Alternatively, the dose can be selected, for example, from the range of 0.001 mg/body to 100,000 mg/body per patient. However, the dose of a pharmaceutical composition of the present invention is not limited to these doses.

The pharmaceutical compositions of the present invention can be formulated according to conventional methods (for example, Remington's Pharmaceutical Science, latest edition, Mark Publishing Company, Easton, U.S.A.), and may also contain pharmaceutically acceptable carriers and additives. Examples include, but are not limited to, surfactants, excipients, coloring agents, flavoring agents, preservatives, stabilizers, buffers, suspension agents, isotonic agents, binders, disintegrants, lubricants, fluidity promoting agents, and corrigents, and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methylcellulose, polyvinylacetal diethylaminoacetate, polyvinylpyrrolidone, gelatin, medium-chain triglyceride, polyoxyethylene hardened castor oil 60, saccharose, carboxymethyl cellulose, corn starch, inorganic salt, and such.

In another aspect, the present invention provides a method for allowing an antigen peptide derived from a target cell to be presented on an MHC class I protein of an antigen-presenting cell, which comprises a step of targeting the antigen-presenting cell to the target cell using a multispecific antigen-binding molecule of the present invention.

In another aspect, the present invention provides a method of screening for an antigen peptide derived from a cancer, which comprises a step of targeting an antigen-presenting cell to a target cell using a multispecific antigen-binding molecule of the present invention and a step of allowing an antigen peptide derived from the target cell to be presented on an MHC class I protein of the antigen-presenting cell. In yet another aspect, the present invention provides a method of screening for an antigen peptide derived from a target cell, which comprises a step targeting an antigen-presenting cell to a target cell using a multispecific antigen-binding molecule of the present invention and a step of detecting an antigen peptide derived from the target cell which has been presented on the MHC class I protein of the antigen presenting cell, which method may include a step of identifying the detected antigen peptide. By using the peptide obtained by the screening method, cytotoxic T cells specific to the MHC/peptide complex containing the peptide can be induced.

Examples of methods for detecting a target cell-derived antigen peptide presented on an MHC class I protein of an antigen presenting cell include a method of evaluating whether the antigen presenting cell can activate a T cell having a TCR reactive to an arbitrary peptide antigen-MHC complex (for example, a system using OT-I mice (having OVA-reactive T cells) described in the Examples section of the present specification), a method of detecting/measuring by FACS using an antibody that specifically detects an arbitrary antigen peptide-MHC complex, a method of directly detecting/measuring by MS the antigen peptide presented on MHC, and such. Methods for detecting/measuring T cell activation include a method of detecting/measuring by ELISA or FACS cytokines released upon activation, a method of detecting/measuring an activation marker by FACS, a method of detecting/measuring an increase in cell number, and the like.

As a method for identifying the detected antigen peptide, for example, the following method is mentioned as an example of a method for identifying the antigen peptide presented by human antigen-presenting cells. Meanwhile, it is also possible to identify antigen peptides presented by mouse antigen-presenting cells by performing a similar experiment using antimouse MHC antibody beads instead of the anti-HLA-DR beads described in the following example method.

### (1) Generation of anti-HLA-DR beads

1. Anti-HLA-DR antibody G46-6 (BD Biosciences, Cat.: 555809) with a final concentration of 1 mg/mL are immobilized onto CNBr-activated Sepharose beads (GE Healthcare, Cat.: 17-0430-01) to prepare anti-HLA-DR antibody-immobilized beads.
2. The anti-HLA-DR antibody-immobilized beads are stored in PBS (Wako, Cat.:041-20211) containing 0.02% sodium azide (Wako, Cat.:190-14901).

### (2) Nanoscale purification of HLA-DR-peptide complexes

1. An ultrapure water (Wako, Cat.:210-01303) solution is prepared by adding 20mM Tris (SIGMA, Cat.:T1503-1KG) and 5mM MgCl₂ (MERCK, Cat.:1.05833.0250) and adjusting pH to 7.8 using HCl (MERCK, Cat.:1.00316.1000) followed by adding thereto 10% TritonX-100 (Roche Diag, Cat.:11332481001) at 1/10 folds, a protease inhibitor mix (a mixture of 11.6 mg/mL PMSF (Nakalai, Cat. :27327-94), 1.7 mg/mL pepstatin A (SIGMA, Cat.:P4265-25MG), 1.7 mg/mL chymostatin (Roche Diag, Cat.:11004638001), 0.8 mg/mL leupeptin (SIGMA, Cat.:L9783 -25MG), and 133 mg/mL sodium azide (Wako, Cat.: 190-14901)) at 17/5000 folds to prepare a lysis buffer.
2. Ten times volume of the lysis buffer is added to a pellet of antigen-presenting cells (e.g. dendritic cells) while cooling on ice, and the resulting mixture is shaken using a Thermomixer Confort (Eppendorf) at 1100 rpm for 1 hour at 4°C to obtain a lysate.
3. Spin down is carried out at 14000 rpm for 10 minutes at 4°C to separate the lysate from cell debris and cell nuclei.
4. A 5-10 µL portion of anti-HLA-DR antibody-immobilized beads are added to 100 µL of the lysate, and the resulting mixture is shaken with a horizontal shaker at 1100 rpm, 4°C, overnight to bind HLA-DR-peptide complexes in the lysate to the anti-HLA-DR antibody-immobilized beads.
5. After spinning down the HLA-DR-peptide complexes bound to the anti-HLA-DR antibody-immobilized beads at 3000 rpm for 1 minute at 4° C, washing is carried out once with 500 µL of the lysis buffer, and twice with 500 µL of PBS containing 0.1% Zwittergent 3-12 (Calbiochem, Cat.:693015).

### (3) Elution of HLA-DR-related peptides

1. The HLA-DR-peptide complexes bound to the HLA-DR antibody-immobilized beads are suspended in 400 µL of ultrapure water. The resulting suspension is transferred to Ultrafree-MC filter (Durapore PVDF, 0.22um) (Millipore) and spinned down at 14000 rpm for 10 seconds at 4°C.
2. The ultrapure water that has dropped to the bottom of the tube is removed, 400 µL of ultrapure water is added onto the filter, and spin down is performed at 14000 rpm for 10-30 seconds at 4°C. This washing operation is repeated 10 times.
3. A 60 uL portion of ultrapure water containing 0.1% trifluoracetic acid (Thermo Fisher Scientific, Cat.: 28904) is added and the resulting mixture is incubated at 37°C for 30 minutes to elute a peptide mixture from the HLA-DR-peptide complexes. Then, spin down is carried out at 14000 rpm for 3 minutes at 18° C, and the eluted peptide mixture is dried with vacuum centrifuge 5305C (Eppendorf).

### (4) Peptide sequence analysis by ion trap MS/MS mass spectrometry

1. The dried peptide mixture is re-dissolved in 15 µL of ultrapure water containing 2% acetonitrile (Wako, Cat.:018-19853), 0.5% acetic acid (MERCK, Cat.:1.00066.0250), 1% formic acid (MERCK, Cat.:1.11670.1000), followed by injecting 5 µL thereof into nano-LC Ultimate 3000 RSLC nano system (Dionex) connected to MS. The LC analysis can be carried out under the conditions that are those described in EP1715343A1 or similar conditions known to those skilled in the art, using a column packed with a combination of a reversed phase material and an ion exchange material or a column packed with a reversed phase material alone, and using an appropriate buffer solution. An HPLC column is connected to an Orbitrap Elite (Thermo) equipped with a nano-LC electrospray ionization source and full scan accurate mass spectrometry and MS-MS mass spectrometry are performed according to the manufacturer's protocol.
2. Sequence analysis of peptides is performed by the SEQUEST algorithm.

Furthermore, the present invention provides methods of inducing damage to cells expressing a certain cancer-specific antigen or to tumor tissues containing cells expressing the cancer-specific antigen and methods for suppressing proliferation of these cells or these tumor tissues, by contacting cells that express the cancer-specific antigen with a multispecific antigen-binding molecule of the present invention which binds to the cancer-specific antigen or with a multispecific antigen-binding molecule of the present invention and an antigen presenting cell activator. The cells bound by an antigen-binding molecule of the present invention that binds to the cancer-specific antigen are not particularly limited as long as they are cells that express the cancer-specific antigens. Specifically, suitable examples of the preferred cancer antigen-expressing cells of the present invention are cells of ovarian cancer, prostate cancer, breast cancer, uterine cancer, hepatic cancer, lung cancer, pancreatic cancer, gastric cancer, bladder cancer, and colorectal cancer.

"Contact" in the present invention is carried out, for example, by administering a multispecific antigen-binding molecule of the present invention that binds to a cancer antigen to a non-human animal transplanted with cells expressing the cancer-specific antigen into the body, or to an animal having cancer cells that intrinsically express the cancer-specific antigen. The method of administration may be either oral or parenteral. In a particularly preferred embodiment, the method of administration is a parenteral administration. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention, a composition for inducing an antitumor immune response, a pharmaceutical composition for inducing cytotoxicity, a cell proliferation suppressor, and an anticancer agent can be administered systemically or locally, for example, through administration by injection. The method of administration can be selected appropriately according to the age and symptoms of test animals. When administered as an aqueous solution, an aqueous solution containing purely an antigen-binding molecule of the present invention alone may be used, or a solution containing surfactants, excipients, coloring agents, perfumes, preservatives, stabilizers, buffers, suspending agents, isotonization agents, binders, disintegrants, lubricants, fluidity promoting agents, flavoring agents, and such may be used. The dose can be selected, for example, from the range of 0.0001 mg to 1000 mg per kg body weight for a single administration. Alternatively, for example, the dose can be selected from the range of 0.001 mg/body to 100000 mg/body per patient. However, the dose of an antigen-binding molecule of the present invention is not limited to these doses.

The following method is suitably used as a method for evaluating or measuring cytotoxicity induced against cells expressing a cancer-specific antigen bound by a cancer specific antigen-binding domain constituting a multispecific antigen-binding molecule of the present invention, through the contact with the antigen-binding molecule. Examples of a method for evaluating or measuring the cytotoxic activity *in vitro* include methods for measuring cytotoxic T cell activity, and such. Whether an antigen-binding molecule of the present invention has T cell cytotoxic activity can be measured by known methods (for example, Current protocols in Immunology, Chapter 7. Immunologic studies in humans, Editor, John E. Coligan et al., John Wiley & Sons, Inc., (1993) and the like). For activity measurements, an antigen-binding molecule with an antigen-binding domain that binds to an antigen which differs from the antigen bound by that of the present invention and is not expressed in the cells used for the examination can be used as a control and in the same manner as the antigen-binding molecule of the present invention, and the activity can be determined to be present when the antigen-binding molecule of the present invention shows a stronger cytotoxic activity than that of the antigen-binding molecule used as a control.

To evaluate or measure cytotoxic activity *in vivo,* for example, cells expressing an antigen bound by a cancer-specific antigen-binding domain that constitutes a multispecific antigen-binding molecule of the present invention are intradermally or subcutaneously transplanted into a non-human test animal, and then a test antigen-binding molecule is intravenously or intraperitoneally administered daily or with an interval of few days, starting from the day of transplantation or the following day. Tumor size is measured daily and the difference in the change of tumor size can be defined as the cytotoxic activity. In a similar manner to the *in vitro* evaluation, a control antigen-binding molecule is administered, and an antigen-binding molecule of the present invention can be determined as exhibiting cytotoxic activity based on the finding that the tumor size in the group subjected to administration of an antigen-binding molecule of the present invention is significantly smaller than the tumor size in the group subjected to administration of the control antigen-binding molecule.

As a method for evaluating or measuring the suppressive effect on proliferation of cells expressing an antigen bound by a cancer-specific antigen-binding domain that constitutes a multispecific antigen-binding molecule of the present invention through the contact with the antigen-binding molecule, a method of measuring the uptake of isotope-labeled thymidine into cells, or the MTT method may be suitably used. As a method for evaluating or measuring the cell proliferation-suppressing activity *in vivo,* the same method as that described above for evaluating or measuring cytotoxic activity *in vivo* may be suitably used.

The present invention also provides kits for use in the methods of the present invention, which comprise an antigen-binding molecule of the present invention or an antigen-binding molecule produced by a production method of the present invention. Additionally, the kit may include in its package, a pharmaceutically acceptable carrier, solvent, instructions describing the method of use, and the like.

The present invention also relates to an antigen-binding molecule of the present invention or an antigen-binding molecule produced by a production method of the present invention for use in a method of the present invention.

Those skilled in the art will naturally understand that optional combinations of one or more of the embodiments described herein are included in the present invention, as long as they are not technically inconsistent based on the common technical knowledge of those skilled in the art.

All prior art references cited herein are incorporated by reference into this description.

In the following, the present disclosure will be described in more detail with reference to examples. However, the present disclosure may be embodied in various forms and should not be construed as being limited to the examples given herein.

### [Examples]

### [Example 1]

### (1) The concept of allowing antigen-presenting cells to present target cell antigens

The various therapies that target existing antigen presenting cells have the following problems.
1. In the case of a therapeutic method in which antigen-presenting cells cultured *ex vivo* are transferred, it is extremely difficult to culture *ex vivo* cells having a function equivalent to that of antigen-presenting cells existing *in vivo.* In addition, culturing needs equipment and time.
2. In the case of a therapeutic method in which an antigen peptide or mRNA is administered, analysis/preparation of an immunizing antigen is required.

The inventors thought it important to meet the following conditions in order to solve these problems.
1. Targeting antigen-presenting cells existing *in vivo* in their natural state.
2. Promoting phagocytosis of target cells and antigen presentation by antigen-presenting cells.

The inventors devised molecules that crosslink antigen-presenting cells and target cells as pharmaceutical compositions that meet the above conditions.

### (2) Examples of antibodies that crosslink antigen-presenting cells to target cells

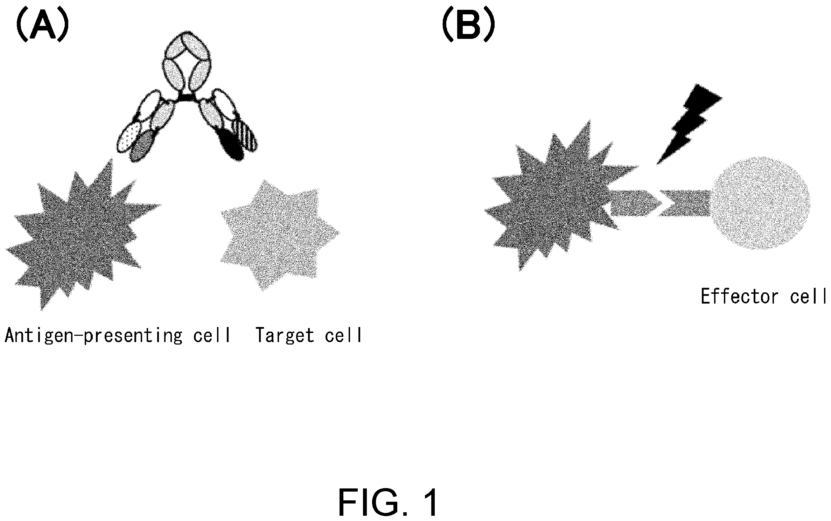

Fig. 1 shows an example of an antibody that crosslinks an antigen-presenting cell to a target cell. A bispecific antibody that recognizes an antigen present on an antigen-presenting cell and an antigen present on a target cell is used to crosslink the antigen-presenting cell to the target cell to promote phagocytosis of the target cell by the antigen-presenting cell. Antigen-presenting cells that phagocytosed target cells can present various antigens including antigens within the target cells to MHC and elicit target cell-reactive immune responses.

### (3) Example methods for enhancing antigen presentation efficiency

For example, among dendritic cells, mature dendritic cells are said to have higher antigen presenting ability than immature dendritic cells (Front Immunol. 2013 Apr 3;4:82). Methods for activating antigen presenting cells include a method of directly activating antigen presenting cells, and a method of activating immune cells other than antigen presenting cells and indirectly activating antigen presenting cells through inflammatory cytokines or such released from these activated cells or damaged dead cells. Therefore, it was thought that it would be possible to increase the efficiency of antigen presentation shown in (1) by using a bispecific antibody that crosslinks antigen-presenting cells and target cells together with a factor that activates antigen-presenting cells (Fig. 2(A)). In addition, it was also thought that it would be possible to increase antigen presentation efficiency by using an antibody made by conjugating a bispecific antibody that crosslinks antigen-presenting cells and target cells with a molecule that directly activates antigen-presenting cells (Fig. 2(B)). Furthermore, it was thought that it would be possible to increase the antigen presentation efficiency shown in (1) by indirectly activating antigen presenting cells by inflammatory cytokines or such released from damaged dead cells through combined use of the bispecific antibody and a bispecific antibody that induces cytotoxic activity by crosslinking a T cell and a cancer cell, for example, as described in WO2012073985 (Fig. 2(C)), or in the form of a conjugate with an antibody or agent that activates cytotoxic T cells (Fig. 2(D)).

### [Example 2] Preparation of bispecific antibodies

CLEC9A is a cross-presenting DC-specific antigen having the function of presenting an engulfed antigen to MHC-I, and GPC3 is known to be expressed on the cell membrane of certain cancer cells (Trends Immunol. 2013 Aug; 34(8): 361-370., European Journal of Cancer Volume 47, Issue 3, February 2011, Pages 333-338). It was thought that, when using these antigens for crosslinking dendritic cells to cancer cells, it is possible to facilitate dendritic cells to phagocytose cancer cells and to present antigens held by cancer cells. Therefore, the present inventors produced a bispecific antibody composed of an anti-CLEC9A antibody and an anti-GPC3 antibody. Expression vectors for known anti-CLEC9A antibody 10B4 (heavy chain: 10B4H-mF18mP4dGK (SEQ ID NO: 1), light chain: 10B4L-mk1 (SEQ ID NO: 2)), and anti-GPC3 antibody GCH065 (heavy chain: GCH065-mF18mN4dGK (SEQ ID NO: 3), light chain: L0011-k0a (SEQ ID NO: 4)) were prepared by a method known to those skilled in the art, and these antibodies were expressed using Expi293 (Thermo Fisher Scientific) cells. A negative control antibody (heavy chain: NCHn (SEQ ID NO: 5), light chain: NCL (SEQ ID NO: 6)) was also used. Purification of antibodies from the culture supernatant and preparation of the bispecific antibody were carried out by methods known to those skilled in the art. Hereinafter, the bispecific antibody of the anti-CLEC9A antibody and the anti-GPC3 antibody will be referred to as 10B4//GCH065, and the bispecific antibody of the anti-CLEC9A antibody and negative control antibody will be referred to as 10B4//NC.

### [Example 3] Evaluation of target cell phagocytosis efficiency

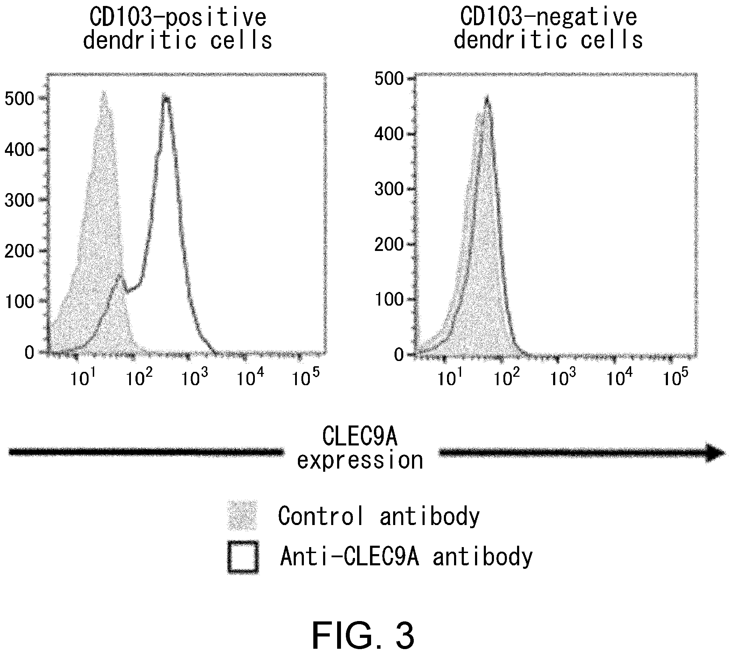

The antibodies prepared in Example 2 were used to evaluate whether the uptake of cancer cells by dendritic cells is promoted by crosslinking the dendritic cells and the cancer cells. The target cancer cells used were LL/2 (LLC1) (CRL-1642, ATCC) transfectant cells (hereinafter LLC1/hGPC3/OVA) that express human GPC3 (hGPC3) and ovalbumin (OVA). The dendritic cells used for the evaluation were BMDC (bone marrow dendritic cells) induced from bone marrow of C57BL/6NCrl mice (female, 6-8 weeks old, Charles River) by the method described previously (Blood 2014 124:3081-3091). The antigen expression in the induced BMDC was stained by a method known to those skilled in the art using the fluorescence-labeled antibodies for flow cytometry shown in Table 1, and the results of CLEC9A expression level evaluation using Fortessa (BD Biosciences) are shown in Fig. 3. As shown in Fig. 3, CD103-positive dendritic cells were CLEC9A-positive and the CD103-negative dendritic cell population was CLEC9A-negative.

**[Table 1]**

| Target antigen | Antibody clone | Label | Manufacturer | Catalog No. |
|---|---|---|---|---|
| CD11c | HL3 | BUV737 | BD | 564986 |
| CD103 | M290 | BV786 | BD | 564322 |
| Clec9A | 7H11 | PE | Biolegend | 143504 |
| Rat lgG1, κ | RTK2071 | | Biolegend | 400408 |
| CD45 | 30-F11 | APC-Cy7 | BioLenged | 103116 |

Target cell phagocytosis efficiency was evaluated using the above cells. First, the target cells LLC1/hGPC3/OVA were labeled using PKH26 Red Fluorescent Cell Linker Kit for General Cell Membrane Labeling (PKH26GL, SIGMA). Next, the labeled cells were frozen in liquid nitrogen and then thawed at 37° C. This operation was carried out twice.

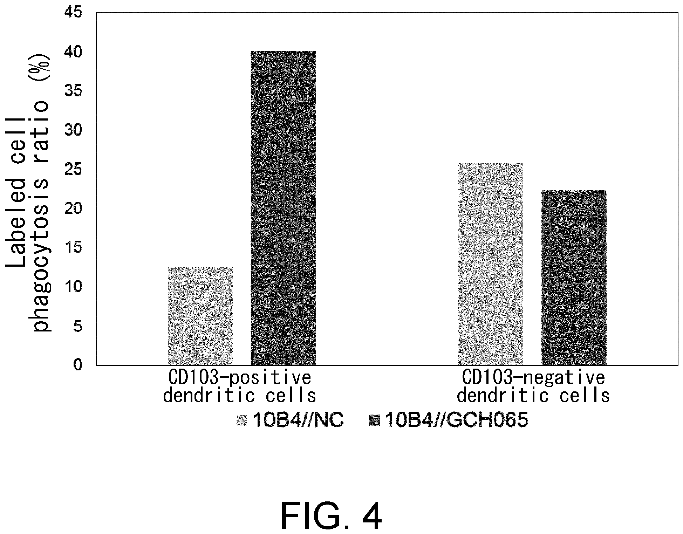

Target cells treated as above, each antibody, and BMDC were added to low-adsorption flat bottom 96 well plates (Cat:3474, Corning) at 2.0 x 10⁴ cells/well, 1 µg/well and 2.0 x 10⁴ cells/well, respectively, and reacted for 1 hour. The reaction was carried out under the conditions of 5% carbon dioxide gas and 37°C. After reacting for 1 hour, the fluorescence-labeled antibodies for flow cytometry shown in Table 2 were used to perform staining by a method known to those skilled in the art, and measured with Fortessa (BD Biosciences). What percentage of each of the CLEC9A-positive dendritic cell (CD103-positive dendritic cell) population and the CLEC9A-negative dendritic cell (CD103-negative dendritic cell) population phagocytosed the labeled cells was evaluated. The result is shown in Fig. 4. 10B4//GCH065 increased the target cell phagocytosis ratio by CLEC9A positive dendritic cells as compared to the negative control antibody 10B4//NC. On the other hand, it did not affect the target cell ratio by CLEC9A-negative dendritic cells.

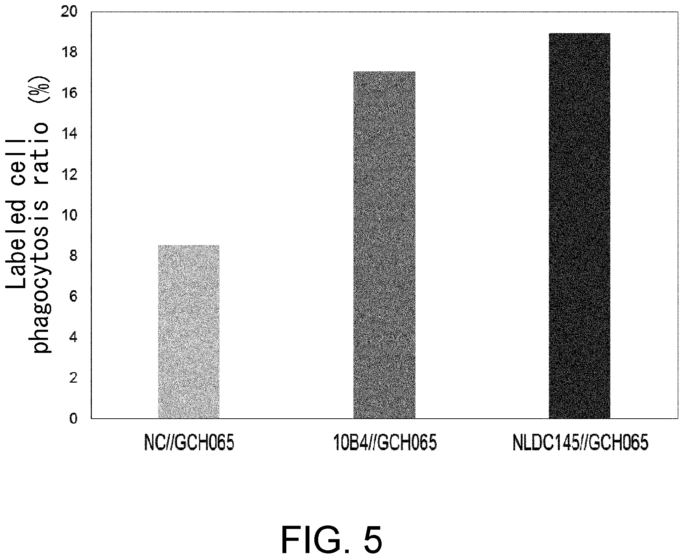

Similarly, the phagocytosis ratio was also enhanced when using the bispecific antibody NLDC145//GCH065 prepared according to the method described in Example 2 using a known antibody NLDC145 (heavy chain: NLDC145VH-mF18mP4dGK (SEQ ID NO: 7), light chain: NLDC145VL-mk1 (SEQ ID NO: 8)), which binds to another antigen DEC205 on CD103-positive dendritic cells, and GCH065 (heavy chain: GCH065-mF18mN4dGK (SEQ ID NO: 3), light chain: L0011-k0a (SEQ ID NO: 4)), which recognizes the target cell antigen GPC3, compared to the bispecific antibody NC//GCH065, which was made using a negative control antibody (heavy chain NCHp (SEQ ID NO: 9), light chain NCL (SEQ ID NO: 6)) and anti-GPC3 antibody GCH065 (heavy chain GCH065-mF18mN4dGK (SEQ ID NO: 3), light chain L0011-k0a (SEQ ID NO: 4)). Test conditions were: target cells LLC1/hGPC3/OVA 5.0 × 10⁴ cells/well, BMDC 2.0 × 10⁴ cells/well, and reaction time: 30 minutes. The results are shown in Fig. 5.

From the above, it was shown that bispecific antibodies that recognize a target antigen and a dendritic cell antigen can induce phagocytosis of target cells by dendritic cells.

**[Table 2]**

| Target antigen | Antibody clone | Label | Manufacturer | Catalog No. |
|---|---|---|---|---|
| CD11c | HL3 | BUV737 | BD | 564986 |
| CD103 | M290 | BV786 | BD | 564322 |
| CD45 | 30-F11 | APC-Cy7 | BioLenged | 103116 |

### [Example 4] Evaluation of antigen presentation efficiency

The bispecific antibody prepared in Example 2 was used to evaluate whether the antigen-presenting cells that phagocytosed the cells could present intrinsic antigens of the target cells and activate immune cells. As immune cells, CD8⁺ T cells isolated with the CD8a⁺T Isolation kit (Cat: 130-104-075, Miltenyi Biotec) from the spleen of OT-I mice (Stock No: 003831, The Jackson Laboratory) having CD8⁺ T cells recognizing the OVA peptide (257-264)-MHC class I complex were used.

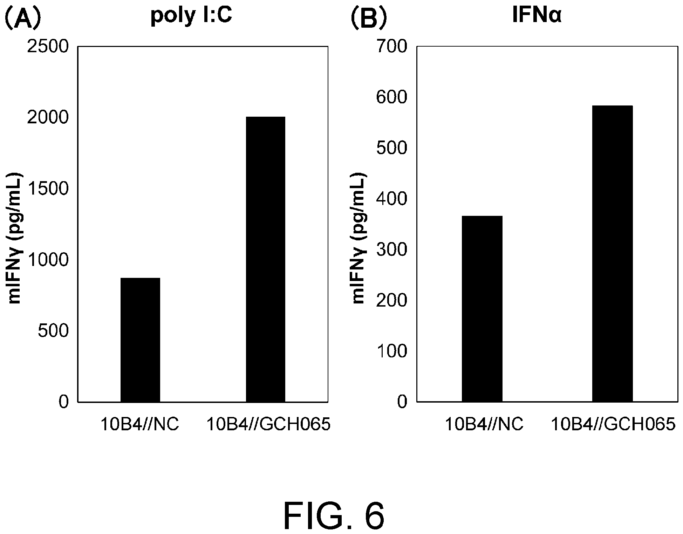

To a low adsorption flat-bottom 96-well-plate (Cat: 3474, Corning) were added target cells LLC1/hGPC3/OVA at 5.0 x 10⁴ cells/well, each antibody at 1 µg/well, CD8⁺ T cells at 1.0 x 10⁵ cells/well, BMDC at 1.0 × 10⁵ cells/well, and poly I:C (Cat: P1530, SIGMA) or IFNα (Cat: 752806, Biolegend) at 20 µg/well or 10000 U/well, respectively, followed by reaction for 48 hours. The reaction was carried out under the conditions of 5% carbon dioxide gas and 37°C. After the reaction was completed, the supernatant was collected, and the amount of mouse IFNγ (mIFNy) in the supernatant was measured using Mouse IFN-gamma DuoSet ELISA (R&D, Cat: DY485). As shown in Figs. 6(A) and 6(B), it was confirmed that the 10B4//GCH065 antibody promotes the ability of dendritic cells to present target cell intracellular antigens. These results indicate that it is possible to induce cross-presentation of antigens within target cells by pMHC-I, by causing dendritic cells to phagocytose the target cells through the use of a bispecific antibody that recognizes a target antigen and a dendritic cell antigen.

### [Example 5] Antitumor effect of the bispecific antibodies

It was evaluated whether an antitumor effect could be induced, using subcutaneous transplantation model mice and the bispecific antibody 10B4//GCH065 composed of anti-CLEC9A antibody 10B4 (heavy chain 10B4H-mF18mP4dGK (SEQ ID NO: 1), light chain 10B4L-mk1 (SEQ ID NO: 2)) and anti-GPC3 antibody GCH065 (heavy chain GCH065-mF18mN4dGK (SEQ ID NO: 3), light chain L0011-k0a (SEQ ID NO: 4)), and the bispecific antibody NC//GCH065 made using a negative control antibody (heavy chain NCHp (SEQ ID NO: 9), light chain NCL (SEQ ID NO: 6)) and anti-GPC3 antibody GCH065 (heavy chain GCH065-mF18mN4dGK (SEQ ID NO: 3), light chain L0011-k0a (SEQ ID NO: 4)) according to the method described in Example 2. First, the target cells, LLC1/hGPC3/OVA, were transplanted to C57BL/6NCrl mice (female, 8 weeks old, Charles River) at 2 × 10⁶ cells each. The tumor diameter and body weight were measured 7 days after transplantation, and randomization was performed based on the results, followed by dividing the mice into 2 groups (6 animals/group). Then, in each randomized group, agents were administered on the 8th and 11th days after transplantation as described below.
Group 1: NC//GCH065 5 mg/kg + poly I:C 50 µg
Group 2: 10B4//GCH065 5 mg/kg + poly I:C 50 µg

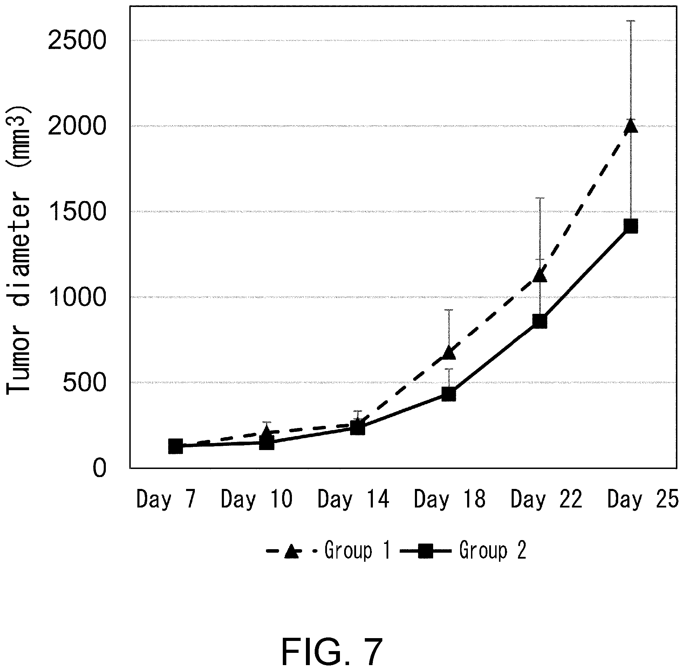

Each antibody was administered intravenously, and poly I:C (tlrl-pic, InvivoGen) was administered intratumorally. The tumor diameter was measured twice a week. A graph of change in tumor diameter is shown in Fig. 7. As shown in Fig. 7, it was confirmed that an antitumor effect was induced by the bispecific antibody that recognizes the target antigen and the dendritic cell antigen.

### [Example 6] Example of activation of antigen-presenting cells

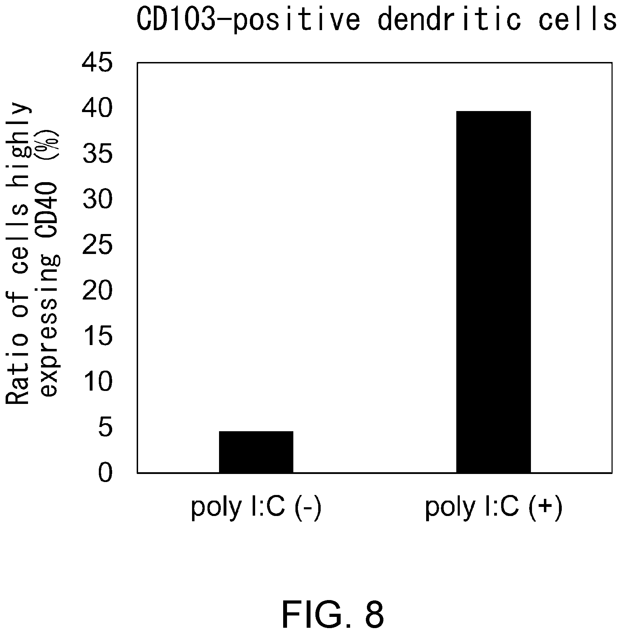

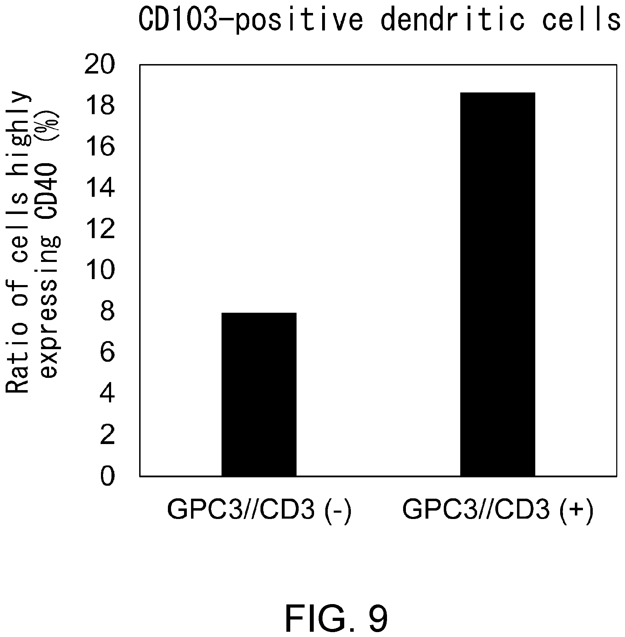

It is thought that antigen presenting efficiency can be increased by activating antigen presenting cells as shown in Example 1 (3). There are two methods for this: a method in which antigen-presenting cells are directly activated; and a method in which immune cells other than antigen-presenting cells are activated and antigen-presenting cells are indirectly activated through inflammatory cytokines released from the activated cells or damaged dead cells. Examples thereof are shown in Figs. 8 and 9.

In Fig. 8, target cells LLC1/GPC3/OVA 7.5×10³ cells/well and BMDC 7.5×10⁴ cells/well were seeded into a low-adsorption flat bottom 96 well plate (Cat: 3474, Corning), and then poly I:C (Cat: P1530, SIGMA) was added at 20 µg/well, followed by reaction for 48 hours. The reaction was carried out under the conditions of 5% carbon dioxide gas and 37°C. After reacting for 48 hours, the fluorescence-labeled antibodies for flow cytometry shown in Table 3 were used for staining by a method known to those skilled in the art, and the CD40 expression level of the CD103-positive cell population was measured using Fortessa (BD Biosciences). As shown in Fig. 8, the expression level of CD40 which is a costimulatory molecule was increased by the addition of poly I:C.

**[Table 3]**

| Target antigen | Antibody clone | Label | Manufacturer | Catalog No. |
|---|---|---|---|---|
| CD3e | 145-2C11 | BUV395 | BD | 563565 |
| CD40 | 3/23 | BV786 | BD | 740891 |
| Rat IgG2a, *κ* | R35-95 | | | 563335 |
| | Zombie Aqua | | Biolegend | 423102 |
| CD45 | 30-F11 | APC-Cy7 | BioLenged | 103116 |
| CD11c | HL3 | A700 | BD | 560583 |
| CD103 | M290 | APC | BD | 562772 |

In Fig. 9, after seeding target cells LLC1/GPC3/OVA 7.5×10³ cells/well, BMDC 7.5×10⁴ cells/well, and T cells 7.5×10⁴ cells/well into a low-adsorption flat-bottom 96 well plate (Cat: 3474, Corning), 0.2 µg of the bispecific antibody GPC3//CD3 antibody, which was prepared by the method described in Example 2 using a known anti-GPC3 antibody H0000-mF18mN4/GL4-mk1 (heavy chain H0000-mF18mN4 (SEQ ID NO: 10), light chain GL4-mk1 (SEQ ID NO: 11)) and the anti-CD3 antibody 2C11VH-mF18mP4/2C11VL-mk1 (heavy chain 2C11VH-mF18mP4 (SEQ ID NO: 12), light chain 2C11VL-mk1 (SEQ ID NO: 13)), was added thereto, followed by reaction for 48 hours. T cells were isolated from spleen cells of C57BL/6NCrl mice (Charles River) using the Pan T Cell Isolation Kit II (Cat: 130-095-130, Miltenyi Biotec). The reaction was carried out under the conditions of 5% carbon dioxide gas and 37°C. Target cells and T cells are crosslinked by GPC3//CD3 antibody to activate T cells (Science Translational Medicine 04 Oct 2017: Vol. 9, Issue 410, eaal4291). After reacting for 48 hours, the fluorescence-labeled antibodies for flow cytometry shown in Table 3 were used for staining by a method known to those skilled in the art, and the CD40 expression level of the CD103-positive cell population was measured using Fortessa (BD Biosciences). As shown in Fig. 9, co-culture with T cells activated by the GPC3//CD3 antibody increased the CD40 expression level on dendritic cells, indicating that dendritic cells can be activated.

### [Example 7] Evaluation of phagocytosis efficiency of hEpCAM-positive target cells

The phagocytosis efficiency of hEpCAM-positive target cells was evaluated using the same system as in Example 3. As target cells, Colon 38 (MC38) (Japanese Foundation For Cancer Research) expressing human EpCAM (hEpCAM) transfectant cells (hereinafter MC38/hEpCAM) were used. The heavy chain and light chain variable regions of the known anti-hEpCAM antibody described in WO2010142990A1 were used.

First, the target cells MC38/hEpCAM were labeled using PKH26 Red Fluorescent Cell Linker Kit for General Cell Membrane Labeling (PKH26GL, SIGMA). Next, the labeled cells were frozen in liquid nitrogen and then thawed at 37° C. This operation was done twice.

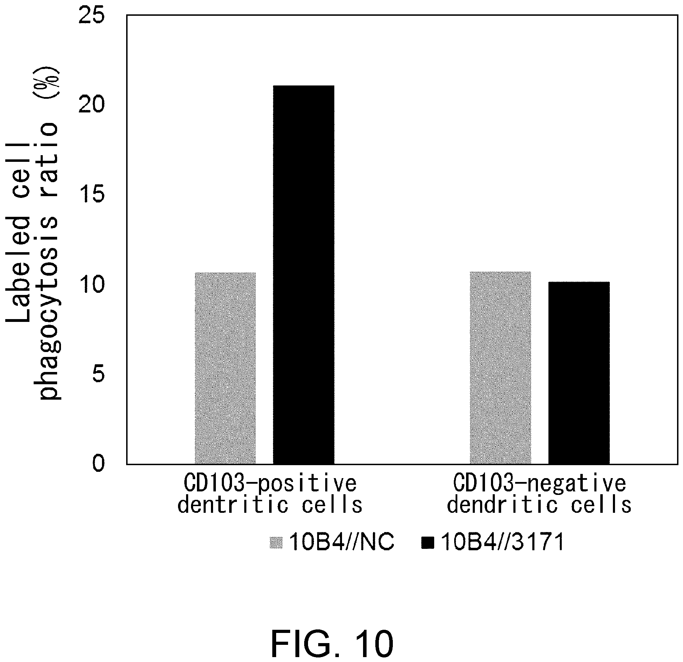

To a low-adsorption flat bottom 96 well plate (Cat: 3474, Corning) were added target cells that had been subjected to the above operation at 5.0 x 10⁴ cells/well, each antibody at 1 µg/well and BMDC at 5.0 x 10⁴ cells/well, followed by reaction for 1 hour at 200 µl/well. The reaction was carried out under the conditions of 5% carbon dioxide gas and 37°C. After reacting for 1 hour, the fluorescence-labeled antibodies for flow cytometry shown in Table 2 were used to perform staining by a method known to those skilled in the art, and measurement was carried out using Fortessa (BD Biosciences). It was evaluated as to what percentage each of the CLEC9A- positive dendritic cell (CD103-positive dendritic cell) population and the CLEC9A-negative dendritic cell (CD103-negative dendritic cell) population phagocytosed the labeled cells. The result is shown in Fig. 10. The bispecific antibody 10B4//3171 prepared by the method described in Example 2 using anti-CLEC9A antibody 10B4 (heavy chain 10B4H-mF18mP4dGK (SEQ ID NO: 1), light chain 10B4L-mk1 (SEQ ID NO: 2)) and anti-hEpCAM-binding antibody 3171 (heavy chain 3171H-mF18mN4dGK (SEQ ID NO: 54)), light chain 3171L-KTO (SEQ ID NO: 55)) increased the ratio of target cell phagocytosis by CLEC9A-positive dendritic cells as compared to the negative control antibody 10B4//NC. On the other hand, it did not affect the target cell ratio by CLEC9A-negative dendritic cells.

### [Example 8] Evaluation of antigen presentation efficiency (NLDC145//GCH065)

The bispecific antibody NLDC145//GCH065 prepared by the method described in Example 2 using known antibody NLDC145 (heavy chain: NLDC145VH-mF18mP4dGK (SEQ ID NO: 7), light chain: NLDC145VL-mk1 (SEQ ID NO: 8)), which binds to DEC205, and GCH065 (heavy chain: GCH065-mF18mN4dGK (SEQ ID NO: 3), light chain: L0011-k0a (SEQ ID NO: 4)), which recognizes the target cell antigen GPC3, was used to evaluate whether antigen presenting cells that phagocytosed cells can present intrinsic antigens of target cells and activate immune cells. As immune cells, as in Example 4, CD8⁺ T cells isolated from the spleen of OT-I mice (Stock No: 003831, The Jackson Laboratory) using the CD8a⁺T Isolation kit (Cat: 130-104-075, Miltenyi Biotec) were used. As target cells, LLC1/hGPC3/OVA which were freeze-thawed 4 times were used.

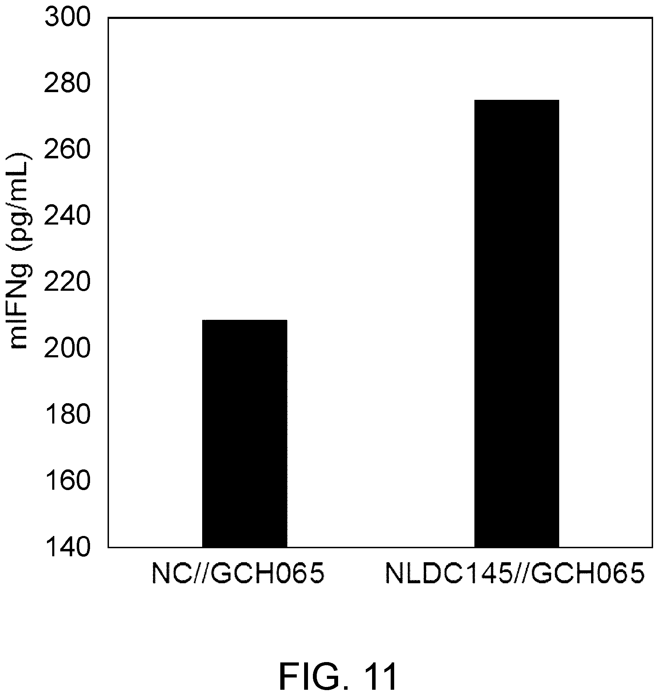

Into a low adsorption flat-bottom 96 well plate (Cat: 3474, Corning) were added target cells LLC1/hGPC3/OVA at 5.0 x 10⁴ cells/well, each antibody at 1 µg/well, CD8⁺ T cells at 1.0 x 10⁵ cells/well, BMDC at 1.0×10⁵ cells/well, and poly I:C (Cat:P1530, SIGMA) at 4 µg/well, followed by reaction for 48 hours at 200 µl/well. The reaction was carried out under the conditions of 5% carbon dioxide gas and 37°C. After the reaction was completed, the supernatant was collected, and the amount of mouse IFNγ (mIFNy) in the supernatant was measured using Mouse IFN-gamma DuoSet ELISA (R&D, Cat: DY485). As shown in Fig. 11, it was confirmed that NLDC145//GCH065 promotes the production of mIFNy and dendritic cell presentation of antigens in the target cells as compared to the negative control antibody NC//GCH065. Therefore, it was shown that NLDC145//GCH065 promotes phagocytosis of target cells by dendritic cells and enhances antigen presentation.

### [Example 9] Evaluation of antigen presentation efficiency (hEpCAM)

The bispecific antibody 10B4//3171 prepared by the method described in Example 2 using CLEC9A antibody 10B4 (heavy chain 10B4H-mF18mP4dGK (SEQ ID NO: 1), light chain 10B4L-mk1 (SEQ ID NO: 2)) and anti-hEpCAM-binding antibody 3171 (heavy chain 3171H-mF18mN4dGK (SEQ ID NO: 54), light chain 3171L-KTO (SEQ ID NO: 55)) was used to evaluate whether antigen presenting cells that phagocytosed cells can present intrinsic antigens of target cells and activate immune cells. As immune cells, as in Example 4, CD8⁺ T cells isolated from the spleen of OT-I mice (Stock No: 003831, The Jackson Laboratory) using the CD8a⁺T Isolation kit (Cat: 130-104-075, Miltenyi Biotec) were used. Target cells used were MC38/hEpCAM/OVA prepared by introducing the Myc-OVA plasmid into MC38/hEpCAM using FuGENE (Cat: E2321A, Promega) to transiently express OVA and freeze-thawing the cells 4 times.

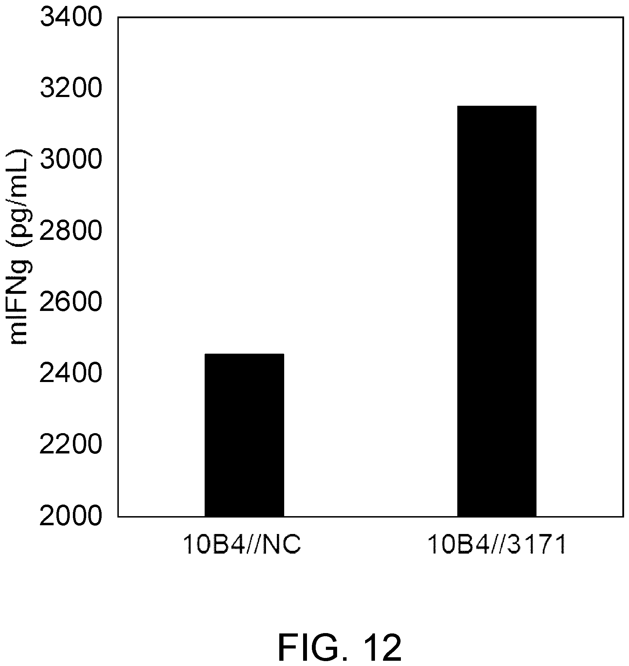

Into a low adsorption flat bottom 96 well plate (Cat: 3474, Corning) were added target cells MC38/hEpCAM/OVA at 1.0 x 10⁴ cells/well, each antibody at 1 µg/well, CD8⁺ T cells at 1.0 x 10⁵ cells/well, BMDC at 1.0×10⁵ cells/well, and poly I:C (Cat: P1530, SIGMA) at 4 µg/well. The mixture was reacted for 48 hours at 200 µl/well. The reaction was carried out under the conditions of 5% carbon dioxide gas and 37°C. After the reaction was completed, the supernatant was collected, and the amount of mouse IFNγ (mIFNy) in the supernatant was measured using Mouse IFN-gamma DuoSet ELISA (R&D, Cat: DY485). As shown in Fig. 12, it was confirmed that 10B4//3171 promotes dendritic cell presentation of antigens in the target cells. Therefore, 10B4//3171 was shown to promote phagocytosis of target cells by dendritic cells and enhance antigen presentation.

**[Table 4]**

| SEQ ID NO: | Name | Explanation |
|---|---|---|
| 1 | 10B4H-mF18mP4dGK | Heavy chain of anti-CLEC9A antibody 10B4 |
| 2 | 10B4L-mk1 | Light chain of anti-CLEC9A antibody 10B4 |
| 3 | GCH065-mF18mN4dGK | Heavy chain of anti-GPC3 antibody GCH065 |
| 4 | L0011-k0a | Light chain of anti-GPC3 antibody GCH065 |
| 5 | IC17HdK-mF18mN4dGK | Heavy chain of the negative control antibody |
| 6 | IC17L-mk1 | Light chain of the negative control antibody |
| 7 | NLDC145VH-mF18mP4dGK | Heavy chain of anti-DEC205 antibody NLDC145 |
| 8 | NLDC145VL-mk1 | Light chain of anti-DEC205 antibody NLDC145 |
| 9 | IC17HdK-mF18mP4dGK | Heavy chain of the negative control antibody |
| 10 | H0000-mF18mN4 | Heavy chain of anti-GPC3 antibody H0000-mF18mN4/GL4-mk1 |
| 11 | GL4-mk1 | Light chain of anti-GPC3 antibody H0000-mF18mN4/GL4-mk1 |
| 12 | 2C11VH-mF18mP4 | Heavy chain of anti-CD3 antibody 2C11VH-mF18mP4/2C11VL-mk1 |
| 13 | 2C11VL-mk1 | Light chain of anti-CD3 antibody 2C11VH-mF18mP4/2C11VL-mk1 |
| 14 | 10B4H-mF18mP4dGK, HCDR1 | Heavy chain CDR1 of anti-CLEC9A antibody 10B4 |
| 15 | 10B4H-mF18mP4dGK, HCDR2 | Heavy chain CDR2 of anti-CLEC9A antibody 10B4 |
| 16 | 10B4H-mF18mP4dGK, HCDR3 | Heavy chain CDR3 of anti-CLEC9A antibody 10B4 |
| 17 | 10B4H-mF18mP4dGK, VH | Heavy chain variable region of anti-CLEC9A antibody 10B4 |
| 18 | 10B4L-mk1, LCDR1 | Light chain CDR1 of anti-CLEC9A antibody 10B4 |
| 19 | 10B4L-mk1, LCDR2 | Light chain CDR2 of anti-CLEC9A antibody 10B4 |
| 20 | 10B4L-mk1, LCDR3 | Light chain CDR3 of anti-CLEC9A antibody 10B4 |
| 21 | 10B4L-mk1, VL | Light chain variable region of anti-CLEC9A antibody 10B4 |
| 22 | GCH065-mF18mN4dGK, HCDR1 | Heavy chain CDR1 of anti-GPC3 antibody GCH065 |
| 23 | GCH065-mF18mN4dGK, HCDR2 | Heavy chain CDR2 of anti-GPC3 antibody GCH065 |
| 24 | GCH065-mF18mN4dGK, HCDR3 | Heavy chain CDR3 of anti-GPC3 antibody GCH065 |
| 25 | GCH065-mF18mN4dGK, VH | Heavy chain variable region of anti-GPC3 antibody GCH065 |
| 26 | L0011-k0a, LCDR1 | Light chain CDR1 of anti-GPC3 antibody GCH065 |
| 27 | L0011-k0a, LCDR2 | Light chain CDR2 of anti-GPC3 antibody GCH065 |
| 28 | L0011-k0a, LCDR3 | Light chain CDR3 of anti-GPC3 antibody GCH065 |
| 29 | L0011-k0a, VL | Light chain variable region of anti-GPC3 antibody GCH065 |
| 30 | NLDC145VH-mF18mP4dGK, HCDR1 | Heavy chain CDR1 of anti-DEC205 antibody NLDC145 |
| 31 | NLDC145VH-mF18mP4dGK, HCDR2 | Heavy chain CDR2 of anti-DEC205 antibody NLDC145 |
| 32 | NLDC145VH-mF18mP4dGK, HCDR3 | Heavy chain CDR3 of anti-DEC205 antibody NLDC145 |
| 33 | NLDC145VH-mF18mP4dGK, VH | Heavy chain variable region of anti-DEC205 antibody NLDC145 |
| 34 | NLDC145VL-mk1, LCDR1 | Light chain CDR1 of anti-DEC205 antibody NLDC145 |
| 35 | NLDC145VL-mk1, LCDR2 | Light chain CDR2 of anti-DEC205 antibody NLDC145 |
| 36 | NLDC145VL-mk1, LCDR3 | Light chain CDR3 of anti-DEC205 antibody NLDC145 |
| 37 | NLDC145VL-mk1, VL | Light chain variable region of anti-DEC205 antibody NLDC145 |
| 38 | 2C11VH-mF18mP4, HCDR1 | Heavy chain CDR1 of anti-CD3 antibody 2C11VH-mF18mP4/2C11VL-mk1 |
| 39 | 2C11VH-mF18mP4, HCDR2 | Heavy chain CDR2 of anti-CD3 antibody 2C11VH-mF18mP4/2C11VL-mk1 |
| 40 | 2C11VH-mF18mP4, HCDR3 | Heavy chain CDR3 of anti-CD3 antibody 2C11VH-mF18mP4/2C11VL-mk1 |
| 41 | 2C11VH-mF18mP4, VH | Heavy chain variable region of anti-CD3 antibody 2C11VH-mF18mP4/2C11VL-mkl |
| 42 | 2C11VL-mk1, LCDR1 | Light chain CDR1 of anti-CD3 antibody 2C11VH-mF18mP4/2C11VL-mk1 |
| 43 | 2C11VL-mkl, LCDR2 | Light chain CDR2 of anti-CD3 antibody 2C11VH-mF18mP4/2C11VL-mk1 |
| 44 | 2C11VL-mk1, LCDR3 | Light chain CDR3 of anti-CD3 antibody 2C11VH-mF18mP4/2C11VL-mk1 |
| 45 | 2C11VL-mk1, VL | Light chain variable region of anti-CD3 antibody 2C11VH-mF18mP4/2C11VL-mk1 |
| 46 | H0000-mF18mN4, HCDR1 | Heavy chain CDR1 of anti-GPC3 antibody H0000-mF18mN4/GL4-mk1 |
| 47 | H0000-mF18mN4, HCDR2 | Heavy chain CDR2 of anti-GPC3 antibody H0000-mF18mN4/GL4-mk1 |
| 48 | H0000-mF18mN4, HCDR3 | Heavy chain CDR3 of anti-GPC3 antibody H0000-mF18mN4/GL4-mk1 |
| 49 | H0000-mF18mN4, VH | Heavy chain variable region of anti-GPC3 antibody H0000-mF18mN4/GL4-mk1 |
| 50 | GL4-mk1, LCDR1 | Light chain CDR1 of anti-GPC3 antibody H0000-mF18mN4/GL4-mk1 |
| 51 | GL4-mk1, LCDR2 | Light chain CDR2 of anti-GPC3 antibody H0000-mF18mN4/GL4-mk1 |
| 52 | GL4-mk1, LCDR3 | Light chain CDR3 of anti-GPC3 antibody H0000-mF18mN4/GL4-mk1 |
| 53 | GL4-mk1, VL | Light chain variable region of anti-GPC3 antibody H0000-mF18mN4/GL4-mk1 |
| 54 | 3171H-mF18mN4dGK | Heavy chain of anti-hEpCAM antibody 3171 |
| 55 | 3171L-KT0 | Light chain of anti-hEpCAM antibody 3171 |
| 56 | 3171H-mF18mN4dGK, HCDR1 | Heavy chain CDR1 of anti-hEpCAM antibody 3171 |
| 57 | 3171H-mF18mN4dGK, HCDR2 | Heavy chain CDR2 of anti-hEpCAM antibody 3171 |
| 58 | 3171H-mF18mN4dGK, HCDR3 | Heavy chain CDR3 of anti-hEpCAM antibody 3171 |
| 59 | 3171H-mF18mN4dGK, VH | Heavy chain variable region of anti-hEpCAM antibody 3171 |
| 60 | 3171L-KT0, LCDR1 | Light chain CDR1 of anti-hEpCAM antibody 3171 |
| 61 | 3171L-KT0, LCDR2 | Light chain CDR2 of anti-hEpCAM antibody 3171 |
| 62 | 3171L-KT0, LCDR3 | Light chain CDR3 of anti-hEpCAM antibody 3171 |
| 63 | 3171L-KT0, VL | Light chain variable region of anti-hEpCAM antibody 3171 |

### [Industrial Applicability]

The multispecific antigen-binding molecules of the present invention can crosslink antigen-presenting cells such as dendritic cells and target cells, promote phagocytosis of the target cells by the antigen-presenting cells, and allow multiple types of tumor antigens derived from the phagocytosed target cells to be presented to T cells. Therefore, the multispecific antigen-binding molecules of the present invention can induce activation of CD8⁺ cytotoxic T cells that target multiple types of tumor antigens derived from phagocytosed target cells, and are a useful means of cancer treatment as compared to conventional cancer immunotherapies that target a single tumor antigen.

## Claims

1. A multispecific antigen-binding molecule comprising a first antigen-binding domain that binds to a first antigen on an antigen-presenting cell and a second antigen-binding domain that binds to a second antigen on a target cell.

2. The multispecific antigen-binding molecule of claim 1, wherein said target cell is a cancer cell.

3. The multispecific antigen binding molecule of claim 1 or 2, which can induce phagocytosis of said target cell by said antigen-presenting cell through the crosslinking of said antigen-presenting cell and said target cell via said multispecific antigen-binding molecule.

4. The multispecific antigen-binding molecule of claim 3, wherein said target cell is an undisrupted target cell.

5. The multispecific antigen-binding molecule of any one of claims 1 to 4, wherein multiple types of antigen peptides derived from said target cell which has been incorporated into the antigen-presenting cell are presented by the antigen-presenting cell.

6. The multispecific antigen-binding molecule of any one of claims 1 to 5, wherein said antigen peptide derived from the target cell and presented on the MHC class I protein of the antigen-presenting cell is derived from an antigen different from the second antigen on the target cell, or is derived from the second antigen on the target cell.

7. The multispecific antigen-binding molecule of any one of claims 1 to 6, wherein said first antigen on the antigen-presenting cell is a dendritic cell surface antigen and is capable of inducing dendritic cell cross-presentation.

8. The multispecific antigen-binding molecule of any one of claims 1 to 7, wherein said antigen on the surface of the antigen-presenting cell is a C-type lectin receptor or an integrin receptor.

9. The multispecific antigen-binding molecule of any one of claims 1 to 8, wherein said first antigen on the antigen-presenting cell is an antigen selected from the group consisting of CLEC9A, DEC205, and CD207.

10. The multispecific antigen-binding molecule of any one of claims 1 to 9, wherein said second antigen on the target cell is a cancer antigen.

11. The multispecific antigen-binding molecule of claim 10, wherein said cancer antigen is an antigen selected from the group consisting of GPC3, IL6R, and EpCAM

12. The multispecific antigen-binding molecule of any one of claims 1 to 11, wherein said antigen-binding molecule is an antibody.

13. A composition for inducing an antitumor immune response, which comprises the multispecific antigen-binding molecule of any one of claims 1 to 12.

14. The composition of claim 13, wherein said induction of the anti-tumor immune response is activation of cytotoxic T lymphocytes (CTLs).

15. A pharmaceutical composition comprising the multispecific antigen-binding molecule of any one of claims 1 to 12.
